# EUROPEAN PATENT SPECIFICATION

(11) **EP 1 610 766 B1**
(45) Date of publication and mention of the grant of the patent: **16.05.2007**
(21) Application number: 04722271.6
(22) Date of filing: 22.03.2004
(51) Int. Cl.: A61K 9/20, C07D 499/00

(54) **AMOXICILLIN TRIHYDRATE**
AMOXICILLINTRIHYDRAT
TRIHYDRATE D'AMOXICILLINE

(30) Priority: 21.03.2003 EP 03100732; 21.03.2003 EP 03100731; 21.03.2003 US 456187 P; 21.03.2003 US 456188 P
(43) Date of publication of application: 04.01.2006
(73) Proprietor: DSM IP Assets B.V., 6411 TE Heerlen (NL)
(72) Inventor: GROENENDAAL, Jan, Willem, 2611 EE DELFT (NL); LEENDERTS, Everardus, Johannus, Anthonius, Maria, 3161 LL RHOON (NL); DOES, VAN DER, Thomas, 3648 AC WILNIS (NL)
(74) Representative: Elkenbracht, Johan Christiaan
(86) International application number: PCT/EP2004/003053
(87) International publication number: WO 2004/082663

(56) References cited:
- WO-A-01/92268

## Description

The present invention relates to a product of amoxicillin trihydrate.

Water in β-lactam antibiotics in solid form may be present in different forms. Water may for instance be present as crystal water. Crystal water refers to water which is integrated in the molecular structure of the β-lactam antibiotic. The amount of crystal water in amoxicillin trihydrate containing 3 molecules of crystal water per molecule of amoxicillin corresponds to about 12.9% crystal water. Free water refers to water that is available for exchange with the atmosphere. The amount of free water does not include the amount of water that is present as crystal water.

The free water content of a sample of a β-lactam antibiotic and the relative humidity of the air that is in contact with the sample influence each other. When a sample of a β-lactam antibiotic is brought in contact with air, exchange of water between said sample and the air will generally take place until an equilibrium situation is established. In the equilibrium situation, the net exchange of water between the sample and the air that is in contact with the sample is zero. The free water content of a sample of a β-lactam antibiotic is typically given for a certain value of the relative humidity in the abovementioned equilibrium situation at a given temperature. This value for the relative humidity is also referred to as equilibrium relative humidity.

The free water content of a sample can be determined by dynamic vapour sorption. The underlying principle is that the weight of the sample is monitored while it is contacted with air having a preconditioned relative humidity. As a result of water take-up or water release the sample weight will change until the equilibrium situation has been established. The sample weight in this equilibrium situation is the sample weight corresponding to the equilibrium relative humidity, the latter being the relative humidity of the preconditioned air with which the sample is contacted. By repeating this procedure for different values of the equilibrium relative humidity, the sample weight can be determined as a function of the equilibrium relative humidity. The free water content of a sample at temperature T can given by ((w_{ERH}-w_{ref})/w_{ref})*100%, wherein W_{ERH} = sample weight, corresponding to equilibrium relative humidity ERH at temperature T and w_{ref} = sample weight, corresponding to a reference value for the equilibrium relative humidity at temperature T, the reference value been chosen such that the free water content at said equilibrium relative humidity is close to zero.

In the application of a β-lactam antibiotic, the presence of free water may be a concern. This may for instance be the case when amoxicillin trihydrate, is mixed with a second pharmaceutically active agent, e.g. clavulanic acid. Therefore, the prior art proposes drying amoxicillin trihydrate to a certain extent.

The water activity, defined as the equilibrium relative humidity divided by 100%, is a method for specifying the extent to which a β-lactam antibiotic has to be dried. The water activity can be measured by bringing a quantity of the sample in a closed chamber having a relatively small volume, measuring the relative humidity as a function of time until the relative humidity has become constant, the latter being the equilibrium relative humidity for that sample. For applications wherein problems associated with water play a role, generally low values for the water activity of the β-lactam antibiotic are specified.

If drying is carried out to an insufficient extent, the problems associated with water remain. If drying is carried out too extensively, the physical properties, such as colour and stability may be impaired. This may for instance be due to the fact that crystal water may be expelled when drying is carried out too extensively.

WO-A-01 92268 discloses compositions comprising amoxillin trihydrate having an equilbrium moisture content of 5% or less.

We surprisingly found a product of amoxicillin trihydrate, having a free water content less than 0.10 wt.%, measured at an equilibrium relative humidity of 30% and at a temperature of 25 °C; and a process for the preparation thereof.

Accordingly a product of amoxicillin trihydrate is provided by the invention giving no or fewer problems with water compared to amoxicillin trihydrate according to the prior art having the same water activity. Moreover, drying may be carried out to a lesser extent, such that properties such as colour and stability are not impaired or impaired to a lesser extent. Due to its lower free water content, the amoxicillin trihydrate, can advantageously be mixed with clavulanic acid or salts thereof, which is known to be very sensitive to moist.

As used herein, the free water content measured at an equilibrium relative humidity of 30% and at a temperature of 25 °C is in particular defined as
((w₃₀-w₁₀)/w₁₀)*100%, wherein
w₃₀ = sample weight, corresponding to an equilibrium relative humidity of 30% at temperature of 25 °C
w₁₀ = sample weight, corresponding to an equilibrium relative humidity of 10% at temperature of 25 °C.

The free water content, including the values for w₃₀ and w₁₀ are preferably determined using dynamic vapour sorption, for instance using a VTI-SGA 100 vapour sorption analyser. Using this technique, adsorptions terms are preferably measured for a sample having a weight of 200 mg by conditioning the air inside the sample chamber at a relative humidity of 10% for 90 minutes, and subsequently increasing the relative humidity with steps of 10%, maintaining the sample at each value for the relative humidity for 90 minutes and taking the sample weight measured after 90 minutes for a value of the relative humidity as the sample weight corresponding to the equilibrium relative humidity.

The product of amoxicillin trihydrate according to the invention has a free water content less than 0.10 wt.%, measured at an equilibrium relative humidity of 30% and at a temperature of 25 °C. Preferably, the product of amoxicillin trihydrate according to the invention has a free water content less than 0.07 wt.%, more preferably less than 0.05 wt.%, measured at an equilibrium relative humidity of 30% and at a temperature of 25 °C. There is no specific upper limit for the free water content. The free water content may for instance be higher than 0.01 wt.%, measured at an equilibrium relative humidity of 30% and at a temperature of 25 °C.

The product of amoxicillin trihydrate according to the invention may be amoxicillin trihydrate in any suitable form, for instance in the form of crystalline powder or granules or mixtures comprising crystalline powder and granules. In a preferred embodiment, the product according to the invention is crystalline amoxicillin trihydrate powder having a free water content of less than 0.1 wt.%, preferably less than 0.07 wt.%, more preferably less than 0.05 wt.%, measured at an equilibrium relative humidity of 30% and at a temperature of 25 °C.

It will be understood that the product of amoxicillin trihydrate may still contain some impurities. Preferably, the product of amoxicillin trihydrate contains at least 90 wt.%, preferably at least 95 wt.%, more preferably at least 98 wt.% of amoxicillin trihydrate. These weight percentages are given relative to the weight of the product. Preferably, the product of amoxicillin trihydrate according to the invention is free of auxiliaries.

As used herein crystalline amoxicillin trihydrate powder refers in particular to a product consisting mainly of crystals of amoxicillin trihydrate. It will be understood that crystals do not refer to aggregates formed by the build-up of crystals, for instance with the aid of a binding agent like water or starch paste, or mechanical force like roller compacting or extrusion. Some unintended formation of aggregates may occur during usual handling, for instance during drying. Aggregates can be seen using optical microscopy applied at a magnification of 140x. As used herein a product consisting mainly of crystals of amoxicilling trihydrate refers in particular to a product comprising at least 70 wt.% crystals of amoxicillin trihydrate, preferably at least 80 wt.%, more preferably at least 90 wt.%, more preferably at least 95 wt.%, more preferably at least 98 wt.%. These percentages can be determined using a combination of air jet sieving and optical microscopy. Air Jet sieving is advantageously carried out using an Alpine Air Jet 200LS-N air jet sieve during 1 minute at 1200 Pa for a sample weight of 10 g. Optical microscopy is advantageously carried out by taking a sample of said fraction of 5 mg, suspending the sample suspending in 4 drops of paraffin oil on a surface on a surface area of 22x40 mm, and using a magnification of 140x.

It will be understood that the amoxicillin trihydrate powder may still contain some impurities. Preferably, the product of amoxicillin trihydrate contains at least 90 wt.%, preferably at least 95 wt.%, more preferably at least 98 wt.% of amoxicillin trihydrate. These weight percentages are given relative to the weight of the crystalline powder. Preferably, the crystalline amoxicillin trihydrate powder according to the invention is free of auxiliaries.

In a preferred embodiment, the product of amoxicillin trihydrate according to the invention, has a water activity of more than 0.05, preferably more than 0.07, preferably more than 0.10, preferably more than 0.15, preferably more than 0.20, preferably more than 0.25, preferably more than 0.30. Increased water contents are advantageous as properties of the amoxicillin trihydrate are not impaired or impaired to a lesser extent, while the amount of free water is still low. There is no specific lower limit for the water activity. In practice, the water activity will generally be less than 0.7, for instance less than 0.6, for instance less than 0.5, although this is not necessary. As used herein these values refer to the water activity at 25 °C.

A preferred method for determining the water activity of a sample is to bring a quantity of the sample in a closed chamber having a relatively small volume, measuring the relative humidity as a function of time until the relative humidity has become constant (for instance after 30 minutes), the latter being the equilibrium relative humidity for that sample. Preferably, a Novasina TH200 Thermoconstanter is used, of which the sample holder has a volume of 12 ml and which is filled with 3 g. of sample.

The d₁₀ and d₅₀ are known ways for indicating a particle size distribution, d₅₀ referring to the value for the particle size such that 50 vol.% of the crystals has a particle size smaller than said value. The d₅₀ is also referred to as average volume-based grain size. Likewise, d₁₀ refers to the value for the particle size such that 10 vol.% of the crystals has a particle size smaller than said value. A preferred way for determining d₁₀ and d₅₀ is laser diffraction, preferably using Malvern equipment.

The d₁₀ and d₅₀ are known ways for indicating a particle size distribution, d₅₀ referring to the value for the particle size such that 50 vol.% of the particles has a particle size smaller than said value. The d₅₀ is also referred to as average volume-based grain size. Likewise, d₁₀ refers to the value for the particle size such that 10 vol.% of the particles has a particle size smaller than said value. A preferred way for determining d₁₀ and d₅₀ is laser diffraction, preferably using Malvern equipment. A suitable apparatus for determining d₁₀ and d₅₀ is a Malvern particle sizer 2600 C obtainable from Malvern Instruments Ltd., Malvern UK, using an objective of f = 300 mm and a beam length of 14.30 mm. A polydisperse analysis model may advantageously be used.

We found that crystalline amoxicillin trihydrate powder according to the invention preferably has an increased d₅₀. The invention therefore also provides crystalline amoxicillin trihydrate powder, preferably having a dso of higher than 10 µm, preferably higher than 20 µm, more preferably higher than 30 µm, more preferably higher than 35 µm, more preferably higher than 40 µm. There is no specific upper limit for the d₅₀. The d₅₀ of the crystalline powder according to the invention may be less than 150 µm, for instance less than 100 µm. The crystalline powder according to the invention preferably has increased d₁₀, preferably higher than 3 µm, preferably higher than 5 µm, more preferably higher than 8 µm, more preferably higher than 10 µm. There is no specific upper limit for the d₁₀ of the crystalline powder according to the invention. The d₁₀ of the crystalline powder according to the invention may be less than 50 µm.

Crystalline amoxicillin trihydrate powder, may be obtained by preparing a solution comprising dissolved amoxicillin, crystallizing the amoxicillin from said solution to form crystals, separating the crystals from said solution, and drying the separated crystals. As used herein the term crystalline powder includes, but is not limited to, the dried product obtained and/or obtainable by this process.

It was found that preparing crystalline powder having decreased free water content for a given equilibrium relative humidity preferably includes carrying out the process, in particular the crystallizing, separating and drying, under such conditions that the dried crystals have an increased particle size, in particular increased d₅₀ and/or d₁₀.

Preferred crystallization conditions include crystallization conditions such that the amoxicillin trihydrate that crystallizes from the solution has increased particle size. This may for instance be achieved by applying a relatively long residence time, relatively low concentrations of amoxicillin in the aqueous solution or using an aqueous solution having high purity. Further preferred conditions are described hereinafter.

The extent of mechanical impact, for instance during separation and/or drying may influence the particle size. Mechanical impact during separation can for instance be achieved during centrifuging. Mechanical impact during drying can for instance be achieved by using a contact dryer, for instance a Vrieco-Nauta contact dryer, or a flash dryer. Mechanical impact can also be achieved by applying pneumatic transport, for instance pneumatic transport of the amoxicillin trihydrate from the separation step to the drying step. A too high extent of mechanical impact is may result in an undesirable decrease of the particle size. Using this insight provided by the invention and by varying the mechanical forces the skilled man is able to find out the conditions how an undesired reduction of particle size can be avoided.

In view of the above, the invention also provides a process for preparing crystalline amoxicillin trihydrate powder, said process comprising: crystallizing amoxicillin trihydrate from a solution; separating the crystals from said solution; drying the separated crystals; wherein the process, preferably the crystallizing, separating and/or drying, is carried out under such conditions that the resulting crystalline powder has a d₅₀ of higher than 10 µm, preferably higher than 20 µm, more preferably higher than 30 µm, in particular higher than 35 µm, more preferably higher than 40 µm. There is no specific upper limit for the d₅₀. The process, preferably the crystallizing, separating and/or drying, may for instance be carried out under such conditions the d₅₀ of the resulting crystalline powder is less than 150 µm, for instance less than 100 µm. Preferably, the process, preferably the crystallizing, separating and/or drying, is carried out under such conditions that the dried crystals have a d₁₀ of higher than 3 µm, preferably higher than 5 µm, more preferably higher than 8 µm, more preferably higher than 10 µm. There is no specific upper limit for d₁₀. The process, preferably the crystallizing, separating and/or drying, may be carried out under such conditions that d₁₀ of the resulting crystalline powder is less than 50 µm.

We found that crystals of amoxicillin trihydrate having decreased free water content may preferably be obtained by applying preferred process conditions described hereinafter.

Preferably, a process for preparing crystalline amoxicillin trihydrate powder according to the invention comprises preparing amoxicillin by reacting 6-amino-penicillanic acid or a salt thereof, with para-hydroxyphenyl glycine in activated form in the presence of an enzyme immobilized on a carrier; forming an aqueous solution containing the amoxicillin, said aqueous solution containing hydrochloric acid; and crystallizing the amoxicillin trihydrate from said aqueous solution.

Preferably, the solution from which the amoxicillin trihydrate is crystallized is an aqueous solution. Any suitable aqueous solution may be used. Suitable aqueous solutions include solutions wherein the weight ration water: organic solvent is between 100 : 0 and 70 : 30, preferably between 100 : 0 and 80 : 20, preferably between 100 : 0 and 90 : 10, preferably between 100 : 0 and 95 : 5, preferably between .100 : 0 and 99 : 1.

Preferably, the solution from which the amoxicillin trihydrate is crystallized contains less than 200 weight parts of protein per 1.000.000 weight parts of amoxicillin (total concentration of amoxicillin, whether or not in dissolved form), preferably less 100 weight parts of protein, more preferably less than 50 weight parts of protein, more preferably less than 35 weight parts of protein.

Preferably, the solution from which the amoxicillin trihydrate is crystallized is an aqueous solution having an amoxicillin concentration (total concentration of amoxicillin, whether or not in dissolved form) of less than 0.6 mol/l, preferably less than 0.5 mol/l, more preferably less than 0.4 mol/l, more preferably less than 0.3 mol/l.

The aqueous solution from which the amoxicillin trihydrate is crystallized, is preferably a solution containing hydrochloric acid or chloride. The aqueous solution from which the amoxicillin trihydrate is crystallized preferably contains between 0.9 and 5 mol of hydrodrochloric acid or chloride per mol amoxicillin (total concentration of amoxicillin, whether or not in dissolved form), preferably between 0.9 and 3 mol hydrochloric acid or chloride per mol amoxicillin, more preferably between 0.9 and 1.5 mol hydrochloric acid or chloride per mol amoxicillin. The aqueous solution from which the amoxicillin is crystallized preferably contains more than 1.0 mol of hydrochloric acid or chloride per mol of amoxicilin.

Preferably, the amoxicillin trihydrate is crystallized from an aqueous solution at a pH of between 2 and 7, preferably between 3 and 6. Preferably, the process comprises crystallizing amoxicillin trihydrate from the aqueous solution in a first step preferably at a pH of between 2 and 5, preferably between 3 and 4, and in a second step at a pH higher than in the first step, preferably between 4 and 7, preferably between 4.5 and 6.

Preferably, amoxicillin trihydrate is crystallized from the aqueous solution at a temperature of between 5 °C and 40 °C, preferably between 10 and 30 °C, more preferably between 15 and 25 °C.

The solution from which the amoxicillin trihydrate is crystallized may be prepared in any suitable way. The aqueous solution containing dissolved amoxicillin may be prepared by dissolving amoxicillin trihydrate. It is possible to add amoxicillin trihydrate to a solution, and to effect dissolution of the added amoxicillin trihydrate. It is also possible to prepare an aqueous suspension by forming crystals of amoxicillin trihydrate in situ in a solution, and effecting dissolution of the crystals of amoxicillin trihydrate in said suspension. In a process for the preparation of amoxicillin, the process preferably comprises preparing an aqueous solution containing dissolved amoxicillin, said aqueous solution having an amoxicillin concentration of less than 0.6 mol/l, preferably less than 0.5 mol/l, more preferably less than 0.4 mol/l, more preferably less than 0.3 mol/l. The process preferably comprises preparing an aqueous solution containing dissolved amoxicillin, said aqueous solution having a pH of between 0 and 1.5, preferably between 0.5 and 1.2. Dissolving amoxicillin, may be carried out in any suitable way, for instance by adding an acid, preferably by adding hydrochloric acid to an aqueous suspension containing crystals of amoxicillin trihydrate. An acid, preferably hydrochloric acid, may be added in an amount of between 0.9 and 5 mol of hydrodrochloric acid per mol amoxicillin, preferably between 0.9 and 3 mol hydrochloric acid per mol amoxicillin, more preferably between 0.9 and 1.5 mol hydrochloric acid per mol amoxicillin. Preferably more than 1.0 mol of hydrochloric acid is added per mol of amoxicillin. In a preferred embodiment, the process comprises keeping the (aqueous) solution or (aqueous) suspension at a pH of less than 1.5, preferably less than 1.2, during a period of less than 60 minutes, preferably less than 30 minutes, more preferably less than 15 minutes, more preferably less than 10 minutes, more preferably less than 8 minutes, as this may improve the purity of the amoxicillin. Preferably, the process comprises mixing the aqueous solution or aqueous suspension with the acid using a fast mixer, for instance a static mixer. This can reduce the time during which the aqueous solution or suspension is kept low pH. Mixing of the acid with the aqueous suspension may be carried out at any suitable temperature, for instance higher than - 5 °C, for instance higher than 5 °C, for instance higher than 10°C, for instance higher than 15 °C, for instance less than 50 °C, for instance less than 40 °C. Preferably, the process comprises filtering the solution prior to said crystallizing. Preferably, the process comprises filtering the aqueous solution containing dissolved amoxicillin, said aqueous solution preferably having having a pH of between 0 and 1.5, preferably between 0.5 and 1.2. The solution may be passed through any suitable filter. Preferably, a filter is used having a pore size of less than 40 µm, preferably less than 20 µm, preferably less than 10 µm, and more preferably less than 5 µm.

Amoxicillin trihydrate may advantageously be crystallized from said aqueous solution by increasing the pH, for instance by adding a base, for instance NaOH.

The crystallization may be carried out batch wise or continuously. When the process is carried out batch wise, addition of seed crystals to the aqueous solution is preferred. Preferably, the crystallization is carried out continuously.

Amoxicillin is preferably prepared by reacting 6-amino-penicilanic acid or derivatives thereof, for instance a salt of 6-amino-penicilanic acid, with an acylating agent selected from para-hydroxyphenyl glycine in activated form in the presence of an enzyme in an aqueous reaction medium. The para-hydroxyphenyl glycine in activated form is preferably an ester or amide of para-hydroxyphenylglycine. Suitable esters include for instance 1 to 4 alkyl esters, for example methyl ester, ethyl ester, n-propyl or isopropyl esters. Glycol esters, for instance an ethylene glycol ester, may also be used. An amide that is unsubstituted in the -CONH₂ group may be used.

The enzyme may be any enzyme having hydrolytic activity (hydrolase). The enzyme may for instance be an acylase, *inter alia* Penicillin G acylase, amidase or esterase. Enzymes may be isolated from various naturally occurring microorganisms, for example fungi and bacteria. Organisms that have been found to produce penicillin acylase are, for example, *Acetobacter, Aeromonas, Alcaligenes, Aphanocladium, Bacillus* sp., *Cephalosporium, Escherichia, Flavobacterium, Kluyvera, Mycoplana, Protaminobacter, Pseudomonas* or *Xanthomonas* species.

Processes for the preparation of amoxicillin in the presence of an enzyme have been described in WO-A-9201061, WO-A-9417800, WO-A-9704086, WO-A-9820120, EP-A-771357, the contents of which are incorporated by reference.

The reaction may be carried out at any suitable pH, preferably at a pH of between 5 and 9, preferably between 5.5 and 8, more preferably between 6 and 7.5. The reaction may be carried out at any suitable temperature, for instance carried out at a temperature of between 0 and 40 °C, preferably between 0 and 30 °C, more preferably between 0 and 15 °C.

The amoxicillin formed may be crystallized under the conditions at which the reaction is carried out. Crystallization of amoxicillin may for instance be effected at a pH of between 5 and 8, preferably between 5.5 and 7.5.

Preferably, the enzyme is an enzyme immobilized on a carrier. Any suitable carrier may be used. Preferably, the carrier comprises a gelling agent and a polymer containing free amino groups. Preferably, the polymer is selected from alginate amine, chitosan, pectin, or polyethylene imine. Preferably, the gelling agent is gelatin. This carrier and the preparation thereof are described in EP-A-222 462 and WO-A-9704086. Prior to immobilization, the isolated enzyme is preferably purified using ion exchange chromatography.

Preferably, the enzyme is an enzyme immobilized on a carrier, and the process preferably comprises separating a product comprising the amoxicillin formed from the immobilized enzyme. Said separating of the product from the immobilized enzyme may be carried out using any suitable method, for instance by using gravity or a screen that is not permeable to the major part of the immobilized enzyme. Preferably, the product separated from the immobilized enzyme contains less than 200 weight parts of protein per 1.000.000 weight parts of the amoxicillin, preferably less 100 weight parts of protein, more preferably less than 50 weight parts of protein, more preferably less than 35 weight parts of protein per 1.000.000 weight parts of the amoxicillin. This is preferably achieved by applying an enzyme sufficiently immobilized on a carrier to avoid separation of small amounts of protein from amoxicillin trihydrate. This embodiment has the advantage that the final amoxicillin trihydrate obtained contains less than 200 weight parts of protein per 1.000.000 weight parts of the amoxicillin, preferably less 100 weight parts of protein, more preferably less than 50 weight parts of protein, more preferably less than 35 weight parts of protein per 1.000.000 weight parts of amoxicillin. The product separated from the immobilized enzyme may be an aqueous solution containing amoxicillin in dissolved form. The product separated from the immobilized enzyme may also be a wet cake. The separated product is preferably an aqueous suspension comprising amoxicillin trihydrate crystals. Preferably, the process comprises dissolving said amoxicillin trihydrate crystals to form an aqueous solution containing dissolved amoxicillin.

The invention also relates to crystalline amoxicillin trihydrate powder obtainable by the process according to the invention.

The product according to the invention can advantageously be used for the preparation of a pharmaceutical composition.

The product of amoxicillin trihydrate according to the invention can advantageously be mixed with pharmaceutically acceptable auxiliaries and/or with a second pharmaceutically active agent. The product of amoxicillin trihydrate,according to the invention can for instance be mixed with between 0 and 50 wt.%, preferably between 0 and 40 wt.%, preferably between 0 and 30 wt.%, more preferably between 0 and 20 wt.%, preferably more than 1 wt.% of auxiliaries, relative to the sum weight of the crystalline powder and the auxiliaries. The product of amoxicillin trihydrate can for instance be mixed with clavulanic acid in salt form, preferably as a potassium salt, the weight ratio amoxicillin : clavulanic acid preferably being between 1:1 and 15:1, preferably between 2:1 and 10:1, preferably between 4:1 and 8:1. These weight ratios are calculated for the anhydrous amoxicillin and clavulanate in acid form. Therefore, the invention also relates to a mixture obtainable by a process comprising mixing the product of amoxicillin trihydrate according to the invention with auxiliaries and/or a second pharmaceutically active agent. The invention also provides a mixture comprising
(i) the product of amoxicillin trihydrate powder according to the invention and (ii) a second pharmaceutically active agent, with or without auxiliaries.

As a second pharmaceutically active agent, clavulanic acid in the form of a salt, preferably clavulanic acid in the form of a potassium salt is preferably used.

As auxiliaries may for instance be used fillers, dry binders, disintegrants, wetting agents, wet binders, lubricants, flow agents and the like. Examples of auxiliaries are lactose, starches, bentonite, calcium carbonate, mannitol, microcrystalline cellulos, polysorbate, sodium lauryl sulphate, carboymethylcelluslose Na, Sodium alginate, magnesium sterarate, silicon dioxid, talc.

In an embodiment, the mixture contains between 0 and 50 wt.%, preferably between 0 and 40 wt.%, preferably between 0 and 30 wt.%, more preferably between 0 and 20 wt.%, preferably more than 1 wt.% of auxiliaries. These weight percentages are given relative to the sum weight of the amoxicillin trihydrate and the auxiliaries.

Preferably the weight ratio amoxicillin : clavulanic acid is between 1:1 and 15:1, preferably between 2:1 and 10:1, preferably between 4:1 and 8:1. These weight ratios are calculated for the anhydrous amoxicillin and clavulanate in acid form.

The product or the mixture according to the invention can advantageously be used for filling a capsule for pharmaceutical use, for instance a gelatine capsule. Therefore, the invention also relates to a capsule containing the product according to the invention or a capsule containing the mixture according to the invention. The product according to the invention or the mixture according to the invention may be fed into a capsule in any suitable way. The invention also relates to the use of the product according to the invention or the mixture according to the invention for filling a capsule.

The invention also provides a process comprising compressing the product according to the invention or compressing the mixture according to the invention to produce compressed products. The compressed products may for instance be granules or tablets. The invention also relates to granules or tablets comprising the product according to the invention in compressed form or comprising the mixture according to the invention in compressed form.

The invention also relates to a process for preparing granules, comprising feeding the crystalline powder according to the invention or the mixture according to the invention, optionally in combination with auxiliaries and/or a second pharmaceutically active agent, to a roller compactor to produce compacts; and milling the compacts to produce granules. The granules produced may advantageously be sieved to obtain a desired particle size distribution. The invention also relates to granules obtainable by this process.

The invention also relates to a process for preparing granules, comprising mixing the crystals according to the invention or the mixture according to the invention with a binding agent, the binding agent for instance being dissolved in a moistening liquid; compacting the crystals whilst moist or dry; granulating the compacts obtained through a sieve. The invention also relates to granules obtainable by this process.

The invention also relates to a process comprising forming a paste from the crystalline powder according to the invention or from the mixture according the invention; kneading the paste at a temperature of 10 °C to 80 °C; extruding the paste in a double-screwed extruder, and, if desired, drying the granules obtained. The invention also relates to granules obtainable by this process.

The invention also relates to a process comprising compressing the granules according to the invention, optionally in mixture with auxiliaries and/or a pharmaceutically active agent to prepare tablets. The invention also relates to tablets obtainable by this process.

We also found that the physical properties of crystalline amoxicillin trihydrate powder can be improved as regards flowability. In a preferred embodiment, the invention as disclosed hereinabove has one or more features of the invention disclosed hereinafter. However, this is not essential.

In an embodiment, the invention disclosed hereinafter has one or more features of the invention disclosed hereinabove. However, this is not essential.

The invention also relates to crystalline amoxicillin trihydrate powder. In addition, the invention relates to a process for preparing crystalline amoxicillin trihydrate powder.

Crystalline powder of a β-lactam antibiotic can be obtained by crystallizing the β-lactam antibiotic from a solution containing the β-lactam antibiotic in dissolved form, separating the crystals obtained, and drying the crystals. In the art, crystals of a β-lactam antibiotic are also referred to as powder.

The known powders have poor flow properties. The particle size is small, and the bulk density is low. This is for instance described in WO-A-9733564 disclosing for instance powder of amoxicillin trihydrate having an average volume-based grain size of 10 µm to 30 µm and a bulk density of 0.15 g/ml to 0.45 g/ml. Due to the poor flow properties of the known powder, the known powder as such is not suitable in applications requiring sufficient flow characteristics, such as for instance filling of capsules.

In order to improve the flow properties, the known powder can be subjected to processes referred to in the art as granulation, compacting, agglomeration, or aggregation to form larger particles having improved flow properties, said larger particles typically having a average volume-based grain size of greater than 100 µm, see for instance WO-A-9733564 and WO-A-9911261. The resulting larger particles having improved flow properties may for instance be used as a filling material for capsules or may be used for the preparation of tablets.

These processes require additional process steps, which is disadvantageous. Moreover, if not properly applied these processes may impair the properties of the antibiotic such as colour, stability. Moreover, we found that the dissolution rate of the resulting product is low.

Surprisingly we found crystalline amoxicillin trihydrate powder having a bulk density higher than 0.45 g/ml; and a process for the preparation thereof.

The crystalline powder according to the invention has improved flow properties, without having to be subjected to processes such as granulation, compacting, agglomeration or aggregation. If it would still be desired to subject the crystalline powder to processes such as granulation, compacting, agglomeration or aggregation, and the like, applying these processes is facilitated due to the improved flow properties of the crystalline powder according to the invention. Moreover, an increased quantity of crystalline powder can be fed into a capsule of a given size.

As used herein crystalline amoxicillin trihydrate powder, refers preferably to a product consisting mainly of crystals of amoxicillin trihydrate. It will be understood that crystals do not refer to aggregates formed by the build-up of crystals, for instance with the aid of a binding agent like water or starch paste, or mechanical force like roller compacting or extrusion. Some unintended formation of aggregates may occur during usual handling, for instance during drying. Aggregates can be seen using optical microscopy applied at a magnification of 140x. As used herein a product consisting mainly of crystals of the β-lactam antibiotic refers preferably to a product comprising at least 70 wt.% crystals of amoxicillin trihydrate, preferably at least 80 wt.%, more preferably at least 90 wt.%, more preferably at least 95 wt.%, more preferably at least 98 wt.%. These percentages can be determined using a combination of air jet sieving and optical microscopy. Air Jet sieving is advantageously carried out using an Alpine Air Jet 200LS-N air jet sieve during 1 minutes at 1200 Pa for a sample weight of 10 g). Optical microscopy is advantageously carried out by taking a sample of 5 mg, suspending the sample in 4 drops of paraffin oil on a surface on a surface area of 22x40 mm, and using a magnification of 140x.

It will be understood that the amoxicillin trihydrate may contain some impurities. The crystalline amoxicillin trihydrate powder according to the invention may for instance contain at least 90 wt.%, preferably at least 95 wt.%, more preferably at least 98 wt.% of amoxicillin trihydrate. These weight percentages are given relative to the weight of the crystalline powder. Preferably, the amoxicillin trihydrate powder is free of auxiliaries.

Preferably, the crystalline powder according to the invention has a bulk density higher than 0.46 g/ml, preferably higher than 0.5 g/ml, more preferably higher than 0.55 g/ml. This further improves the flow properties. Moreover, an increased bulk density is advantageous as crystalline powder may be fed into a certain volume, for instance a capsule. There is no specific upper limit for the bulk density. The bulk density may be less than 0.8 g/ml, for instance less than 0.7 g/ml. The bulk density is preferably determined according to USP 24, method I, (page 1913).

Preferably, the crystalline powder according to the invention has a tapped density higher than 0.6 g/ml, preferably higher than 0.7, more preferably higher than 0.8 g/ml. An increased tapped density improves the flow properties. Moreover, an increased tapped density is advantageous as more products may be fed into a certain volume, for instance a capsule. There is no specific upper limit for the tapped density. The tapped density may be less than 1.2 g/ml, for instance less than 1.1 g/ml, for instance less than 1.0 g/ml. The tapped density is preferably determined according to USP 24, method II, (page 1914).

The invention also relates to crystalline amoxicillin trihydrate powder, having a tapped density higher than 0.6 g/ml, preferably higher than 0.7, more preferably higher than 0.8 g/ml. This crystalline powder has improved flow properties compared to the known powder. The tapped density may be less than 1.2 g/ml, for instance less than 1.1 g/ml, for instance less than 1.0 g/ml.

Preferably, the crystalline powder according to the invention has a bulk densitity and tapped density such that the ratio dₜ/d_{b} is less than 1.7, preferably less than 1.6, preferably less than 1.5, preferably less than 1.45, wherein dₜ = tapped density and d_{b} = bulk density. This results in improved flow ability. There is no specific lower limit for the ratio dₜ/d_{b}. The ratio dₜ/d_{b} may be higher than 1.05, for instance higher than 1.1.

The invention also relates to crystalline amoxicillin trihydrate powder, having a bulk density and tapped density such that the ratio dₜ/d_{b} is less than 1.7, preferably less than 1.6, preferably less than 1.5, preferably less than 1.45. This crystalline powder has improved flow properties compared to the known powder. There is no specific upper limit for the ratio dₜ/d_{b}. The ratio dₜ/d_{b} may be higher than 1.05, for instance higher than 1.1.

Preferably, the crystalline powder according to the invention has a bulk densitity and tapped density such that the compressibility index as defined by ((dₜ - d_{b})/dₜ)*100% is less than 40%, preferably less than 35%, more preferably less than 30%. This results in improved flow ability. There is no specific lower limit for the compressibility index. The compressibility index may for instance be higher than 10%.

The invention also relates to crystalline amoxicillin trihydrate powder having a bulk density and tapped density such that the compressibility index as defined by ((dₜ - d_{b})/dₜ)*100% is less than 40%, preferably less than 35%, more preferably less than 30%. This crystalline powder has improved flow properties compared to the known powder. There is no specific lower limit for the compressibility index. The compressibility index may for instance be higher than 10%.

The d₁₀ and d₅₀ are known ways for indicating a particle size distribution, d₅₀ referring to the value for the particle size such that 50 vol.% of the particles has a particle size smaller than said value. The d₅₀ is also referred to as average volume-based grain size. Likewise, d₁₀ refers to the value for the particle size such that 10 vol.% of the particles has a particle size smaller than said value. A preferred way for determining d₁₀ and d₅₀ is laser diffraction, preferably using Malvern equipment. A suitable apparatus for determining d₁₀ and d₅₀ is a Malvern particle sizer 2600 C obtainable from Malvern Instruments Ltd., Malvern UK, for instance using an objective of f = 300 mm and a beam length of 14.30 mm. A polydisperse analysis model may advantageously be used.

We found that crystalline powder having improved flowability, bulk density and/or tapped density preferably have an increased d₅₀. The invention also provides crystalline amoxicillin trihydrate powder having a d₅₀ of higher than 10 µm, preferably higher than 20 µm, more preferably higher than 30 µm, more preferably higher than 35 µm, more preferably higher than 40 µm. There is no specific upper limit for the d₅₀. The d₅₀ of the crystalline powder according to the invention may be less than 150 µm, for instance less than 100 µm. The crystalline powder according to the invention preferably has increased d₁₀, preferably higher than 3 µm, preferably higher than 5 µm, more preferably higher than 8 µm, more preferably higher than 10 µm. There is no specific upper limit for the d₁₀ of the crystalline powder according to the invention. The d₁₀ of the crystalline powder according to the invention may be less than 50 µm.

The invention also provides a process comprising sieving the crystalline powder according to the invention. This allows the physical properties of the crystalline powder to be improved even further. Preferably, air jet sieving is applied.

The crystalline powder according to the invention can advantageously be used for the preparation of a pharmaceutical composition.

The crystalline powder according to the invention can advantageously be mixed with pharmaceutically acceptable auxiliaries and/or with a second pharmaceutically active agent. The crystalline powder according to the invention can for instance be mixed with between 0 and 50 wt.%, preferably between 0 and 40 wt.%, preferably between 0 and 30 wt.%, more preferably between 0 and 20 wt.%, preferably more than 1 wt.% of auxiliaries, relative to the sum weight of the crystalline powder and the auxiliaries. The crystalline powder according to the invention can for instance be mixed with clavulanic acid in salt form, preferably as a potassium salt, the weight ratio amoxicillin : clavulanic acid preferably being between 1:1 and 15:1, preferably between 2:1 and 10:1, preferably between 4:1 and 8:1. These weight ratios are calculated for the anhydrous amoxicillin and clavulanate in acid form. Therefore, the invention also relates to a mixture obtainable by a process comprising mixing the crystalline powder according to the invention with auxiliaries and/or a second pharmaceutically active agent. The invention also provides a mixture comprising (i) the crystalline powder according to the invention and (ii) auxiliares and/or a second pharmaceutically active agent.

As a second pharmaceutically active agent, clavulanic acid in the form of a salt, preferably clavulanic acid in the form of a potassium salt is preferably used.

As auxiliaries may for instance be used fillers, dry binders, disintegrants, wetting agents, wet binders, lubricants, flow agents and the like. Examples of auxiliaries are lactose, starches, bentonite, calcium carbonate, mannitol, microcrystalline cellulos, polysorbate, sodium lauryl sulphate, carboymethylcelluslose Na, Sodium alginate, magnesium sterarate, silicon dioxid, talc.

Preferably, the mixture contains between 0 and 50 wt.%, preferably between 0 and 40 wt.%, preferably between 0 and 30 wt.%, more preferably between 0 and 20 wt.%, preferably more than 1 wt.% of auxiliaries. These weight percentages are given relative to the sum weight of the amoxicillin trihydrate and the auxiliaries.

Preferably the weight ratio amoxicillin : clavulanic acid is between 1:1 and 15:1, preferably between 2:1 and 10:1, preferably between 4:1 and 8:1. These weight ratios are calculated for the anhydrous amoxicillin and clavulanate in acid form.

The crystalline powder according to the invention can advantageously be used for filling a capsule for pharmaceutical use, for instance a gelatine capsule. Therefore, the invention also relates to a capsule containing the crystalline powder according to the invention or a capsule containing the mixture according to the invention. The crystalline powder according to the invention or the mixture according to the invention may be fed into a capsule in any suitable way. It will be understood by the skilled person that feeding material into a capsule may comprise forming a plug of the material, said plug being loosely comprised of the material. The skilled man will understand that a capsule containing crystalline powder or the mixture according to the invention also encompasses a capsule containing a plug of crystalline powder or a mixture according to the invention. The invention also relates to the use of the crystalline powder according to the invention or the mixture according to the invention for filling a capsule or for the preparation of a tablet.

The invention also relates to a process for filling a capsule, comprising feeding the crystalline powder according to the invention, optionally in combination with auxiliaries and/or a second pharmaceutically active agent, into the capsule. The invention also relates to a process comprising mixing the crystalline powder according to the invention with pharmaceutically acceptable auxiliaries, optionally together with a second pharmaceutically active agent, and feeding the resulting mixture into a capsule.

We found that the improved flow properties of the crystalline amoxicillin trihydrate powder according to the invention facilitate processes such as dry and wet granulation, agglomeration, tablet formation and the like. Therefore, the invention also provides a process comprising compressing the crystalline powder according to the invention or compressing the mixture according to the invention to produce compressed products. The compressed products may for instance be granules or tablets. The invention also relates to granules or tablets comprising the crystalline powder in compressed form or comprising the mixture according to the invention in compressed form.

The invention also relates to a process for preparing granules, comprising feeding the crystalline powder according to the invention or the mixture according to the invention, optionally in combination with auxiliaries and/or a second pharmaceutically active agent, to a roller compactor to produce compacts; and milling the compacts to produce granules. The granules produced may advantageously be sieved to obtain a desired particle size distribution. The invention also relates to granules obtainable by this process.

The invention also relates to a process for preparing granules, comprising mixing the crystalline powder according to the invention or the mixture according to the invention with a binding agent, the binding agent for instance being dissolved in a moistening liquid; compacting the crystals whilst moist or dry; granulating the compacts obtained through a sieve. The invention also relates to granules obtainable by this process.

The invention also relates to a process comprising forming a paste from the crystalline powder according to the invention or from the mixture according the invention; kneading the paste at a temperature of 10 °C to 80 °C; extruding the paste in a double-screwed extruder, and, if desired, drying the granules obtained. The invention also relates to granules obtainable this process.

The invention also relates to a process comprising compressing the granules according to the invention, optionally in mixture with auxiliaries and/or a pharmaceutically active agent to prepare tablets. The invention also relates to tablets obtainable by this process.

The invention also provides a process comprising mixing the crystalline amoxicillin trihydrate powder according to the invention, with amoxicillin in another physical form, preferably granules comprising amoxicillin trihydrate. The invention also provides a mixture comprising (i) amoxicillin trihydrate powder according to the invention and (ii) amoxicillin trihydrate in another physical form, preferably granules comprising amoxicillin trihydrate. In an embodiment, this mixture is auxilary free. In an embodiment, this mixture comprises auxiliaries and/or a second pharmaceutically active agent. The granules comprising amoxicillin trihydrate in the mixture (to be mixed) may be any suitable granules, for instance having a d₅₀ of between 100 and 1000 µm. The d₅₀ of granules is preferably determined by making a sieve analysis. The granules may for instance comprise at least 90 wt.% of amoxicillin trihydrate, preferably at least 95 wt.%, more preferably at least 98 wt.% of amoxicillin trihydrate. Preferably, the granules are free of auxiliaries. The granules may be obtained by any suitable process wherein powder is combined to form granules, for instance by roller compacting, agglomeration, extrusion, aggregation, wet or dry granulation.

In one aspect of the invention, the crystalline powder according to the invention has a high dissolution rate. Preferably, the crystalline powder according to the invention has a T_{85%} of less than 55 minutes, preferably less than 50 minutes, preferably less than 40 minutes, preferably less than 30 minutes, preferably less than 25 minutes, preferably less than 20 minutes. T_{85%} may for instance be above 5 minutes. As used herein T_{85%} refers to period required to effect dissolution of 85 wt.% of a predetermined quantity (500 mg on an anhydrous basis) of the amoxicillin trihydrate in 900 ml water of 37 °C. The determination of T_{85%} is preferably carried out under conditions as defined in USP 27, chapter "amoxicillin capsules", involving the use of apparatus 1 and applying a stirring rate of 100 rpm (USP 27, paragraph 711). Preferably, samples are taken at regular intervals, e.g. each 5 minutes, to determine the quantity of dissolved amoxicillin by UV absorption. Known products of amoxicillin trihydrate having a bulk density higher than 0.45 g/ml, for instance granules of amoxicillin trihydrate having a bulk density higher than 0.45 g/ml, are not found to have such high dissolution rates.

Therefore, the invention also relates to a product of amoxicillin trihydrate, said product having:
(i) a T_{85%} of less than 55 minutes, preferably less than 50 minutes, preferably less than 40 minutes, preferably less than 30 minutes, preferably less than 25 minutes; and
(ii) a bulk density higher than 0.45 g/ml, preferably higher than 0.5 g/ml, more preferably higher than 0.55 g/ml. There is no specific upper limit for the bulk density. The bulk density may be less than 0.8 g/ml, for instance less than 0.7 g/ml. T_{85%} may for instance be above 5 minutes.

The invention also relates to a product amoxicillin trihydrate, said product having
(i) a T_{85%} of less than 55 minutes, preferably less than 50 minutes, preferably less than 40 minutes, preferably less than 30 minutes, preferably less than 25 minutes; and
(ii) a tapped density higher than 0.6 g/ml, preferably higher than 0.7, more preferably higher than 0.8 g/ml. There is no specific upper limit for the tapped density. The tapped density may be less than 1.2 g/ml, for instance less than 1.1 g/ml, for instance less than 1.0 g/ml.

The invention also relates to a product of amoxicillin trihydrate, said product having:
(i) a T_{85%} of less than 55 minutes, preferably less than 50 minutes, preferably less than 40 minutes, preferably less than 30 minutes, preferably less than 25 minutes; and
(ii) a bulk densitity and tapped density such that the ratio dₜ/d_{b} is less than 1.7, preferably less than 1.6, preferably less than 1.5, preferably less than 1.45, wherein dₜ = tapped density and d_{b} = bulk density. This results in improved flow ability. There is no specific lower limit for the ratio dₜ/d_{b}. The ratio dₜ/d_{b} may be higher than 1.05, for instance higher than 1.1.

The invention also relates to amoxicillin trihydrate, said product having
(i) a T_{85%} of less than 55 minutes, preferably less than 50 minutes, preferably less than 40 minutes, preferably less than 30 minutes, preferably less than 25 minutes; and
(ii) a bulk densitity and tapped density such that the compressibility index as defined by ((dₜ - d_{b})/dₜ)*100% is less than 40%, preferably less than 35%, more preferably less than 30%. This results in improved flow ability. There is no specific lower limit for the compressibility index. The compressibility index may for instance be higher than 10%.

In a preferred embodiment, the product of amoxicillin trihydrate according to the invention has abovementioned preferred values for bulk density, tapped density, ratio dₜ/d_{b} and compressibility index mentioned above in combination.

The product of amoxicillin trihydrate according to the invention may be amoxicillin trihydrate in any suitable form. It will be understood that the product of amoxicillin trihydrate may still contain some impurities. Preferably, the product of amoxicillin trihydrate contains at least 90 wt.%, preferably at least 95 wt.%, more preferably at least 98 wt.% of amoxicillin trihydrate. These weight percentages are given relative to the weight of the product. Preferably, the product of amoxicillin trihydrate according to the invention is free of auxiliaries.

In one embodiment the product according to the invention is crystalline amoxicillin trihydrate powder according to the invention. In another embodiment embodiment the product according to the invention is a mixture comprising (i) amoxicillin trihydrate powder, preferably crystalline amoxicillin trihydrate powder according to the invention and (ii) amoxicillin trihydrate in another physical form, preferably granules comprising amoxicillin trihydrate.

The invention also provides a process comprising mixing amoxicillin trihydrate powder, preferably the crystalline amoxicillin trihydrate powder according to the invention, with amoxicillin in another physical form, preferably granules comprising amoxicillin trihydrate to obtain the product according to the invention. The quantities of amoxicillin trihydrate and granules to be mixed can be chosen such that preferred combinations of T_{85%} and the bulk density, tapped density, ratio dₜ/d_{b} and/or compressibility index are achieved. The amoxicillin trihydrate powder in the mixture (to be mixed) may be a powder having for instance a d₅₀ of between 1 and 100 µm. The crystalline amoxicilin trihydrate powder according to the invention is preferred.

The granules comprising amoxicillin trihydrate in the mixture (to be mixed) may be any suitable granules, for instance having a d₅₀ of between 100 and 1000 µm. The d₅₀ of the granules is preferably determined by making a sieve analysis..The granules may for instance comprise at least 90 wt.% of amoxicillin trihydrate, preferably at least 95 wt.%, more preferably at least 98 wt.% of amoxicillin trihydrate. Preferably, the granules are free of auxiliaries. The granules may be obtained by any suitable process wherein powder is combined to form granules, for instance by roller compacting, agglomeration, extrusion, aggregation, wet or dry granulation.

The product according to the invention can advantageously be used for the preparation of a pharmaceutical composition.

The product according to the invention can advantageously be mixed with pharmaceutically acceptable auxiliaries and/or with a second pharmaceutically active agent. The product according to the invention can for instance be mixed with between 0 and 50 wt.%, preferably between 0 and 40 wt.%, preferably between 0 and 30 wt.%, more preferably between 0 and 20 wt.%, preferably more than 1 wt.% of auxiliaries, relative to the sum weight of the product and the auxiliaries. The product according to the invention can for instance be mixed with clavulanic acid in salt form, preferably as a potassium salt, the weight ratio amoxicillin : clavulanic acid preferably being between 1:1 and 15:1, preferably between 2:1 and 10:1, preferably between 4:1 and 8:1. These weight ratios are calculated for the anhydrous amoxicillin and clavulanate in acid form. Therefore, the invention also relates to a mixture obtainable by a process comprising mixing the product according to the invention with auxiliaries and/or a second pharmaceutically active agent. The invention also provides a mixture comprising (i) the product according to the invention and (ii) auxiliares and/or a second pharmaceutically active agent.

As a second pharmaceutically active agent, clavulanic acid in the form of a salt, preferably clavulanic acid in the form of a potassium salt is preferably used.

As auxiliaries may for instance be used fillers, dry binders, disintegrants, wetting agents, wet binders, lubricants, flow agents and the like. Examples of auxiliaries are lactose, starches, bentonite, calcium carbonate, mannitol, microcrystalline cellulos, polysorbate, sodium lauryl sulphate, carboymethylcelluslose Na, Sodium alginate, magnesium sterarate, silicon dioxid, talc.

Preferably, the mixture contains between 0 and 50 wt.%, preferably between 0 and 40 wt.%, preferably between 0 and 30 wt.%, more preferably between 0 and 20 wt.%, preferably more than 1 wt.% of auxiliaries. These weight percentages are given relative to the sum weight of the amoxicillin trihydrate and the auxiliaries.

Preferably the weight ratio amoxicillin : clavulanic acid is between 1:1 and 15:1, preferably between 2:1 and 10:1, preferably between 4:1 and 8:1. These weight ratios are calculated for the anhydrous amoxicillin and clavulanate in acid form.

The product according to the invention can advantageously be used for filling a capsule for pharmaceutical use, for instance a gelatine capsule. Therefore, the invention also relates to a capsule containing the product according to the invention or a capsule containing the mixture according to the invention. The product according to the invention or the mixture according to the invention may be fed into a capsule in any suitable way. It will be understood by the skilled person that feeding material into a capsule may comprise forming a plug of the material, said plug being loosely comprised of the material. The skilled man will understand that a capsule containing product or the mixture according to the invention also encompasses a capsule containing a plug of product or a mixture according to the invention. The invention also relates to the use of the product according to the invention or the mixture according to the invention for filling a capsule or for the preparation of a tablet.

The invention also relates to a process for filling a capsule, comprising feeding the product according to the invention, optionally in combination with auxiliaries and/or a second pharmaceutically active agent, into the capsule. The invention also relates to a process comprising mixing the product according to the invention with pharmaceutically acceptable auxiliaries, optionally together with a second pharmaceutically active agent, and feeding the resulting mixture into a capsule.

We found that the improved flow properties of the product according to the invention facilitate processes such as dry and wet granulation, agglomeration, tablet formation and the like. Therefore, the invention also provides a process comprising compressing the product according to the invention or compressing the mixture according to the invention to produce compressed products. The compressed products may for instance be granules or tablets. The invention also relates to granules or tablets comprising the product according to the invention in compressed form or the mixture according to the invention in compressed form.

The invention also relates to a process for preparing granules, comprising feeding the product according to the invention or the mixture according to the invention, optionally in combination with auxiliaries and/or a second pharmaceutically active agent, to a roller compactor to produce compacts; and milling the compacts to produce granules. The granules produced may advantageously be sieved to obtain a desired particle size distribution. The invention also relates to granules obtainable by this process.

The invention also relates to a process for preparing granules, comprising mixing the product according to the invention or the mixture according to the invention with a binding agent, the binding agent for instance being dissolved in a moistening liquid; compacting the crystals whilst moist or dry; granulating the compacts obtained through a sieve. The invention also relates to granules obtainable by this process.

The invention also relates to a process comprising forming a paste from the product according to the invention or from the mixture according the invention; kneading the paste at a temperature of 10°C to 80 °C; extruding the paste in a double-screwed extruder, and, if desired, drying the granules obtained. The invention also relates to granules obtainable this process.

The invention also relates to a process comprising compressing the granules according to the invention, optionally in mixture with auxiliaries and/or a pharmaceutically active agent to prepare tablets. The invention also relates to tablets obtainable by this process.

Crystalline amoxicillin trihydrate powder may be obtained by preparing a solution comprising dissolved amoxicillin, crystallizing the amoxicillin trihydrate from said solution to form crystals, separating the crystals from said solution, and drying the separated crystals. As used herein the term crystalline powder includes, but is not limited to, the dried product obtained and/or obtainable by this process.

It was surprisingly found that crystalline powder having improved flow properties, in particular the high bulk density and/or high tapped density can be obtained by selecting the crystallizing, separation and/or drying conditions.

It was found that preparing crystalline powder having improved flow properties, in particular high bulk density and/or high tapped density, preferably includes carrying out the process, in particular the crystallizing, separating and drying, under such conditions that the dried crystals have an increased particle size, in particular increased d₅₀ and/or d₁₀.

Preferred crystallization conditions may include a relatively long residence time, relatively low concentrations of amoxicillin in the aqueous solution, relatively low concentrations of protein in the aqueous solution and/or using an aqueous solution having high purity. Further preferred conditions are described hereinafter.

It was further found that, in particular for crystals having an increased size, the extent of mechanical impact, for instance during crystallization, separation and/or drying influences the bulk density and tapped density. If the crystals are subjected to mechanical forces, for instance during drying and/or separating or transport of the crystals, the bulk density and tapped density are surprisingly found to increase compared to the situation where there is no mechanical impact. However, if the mechanical forces are too high, the bulk density and tapped density are found to decrease. Mechanical impact during separating can for instance be achieved during centrifuging. Mechanical impact during drying can for instance be achieved by using a contact dryer, for instance a Vrieco-Nauta contact dryer, or a flash dryer. Mechanical impact can also be achieved by applying pneumatic transport, for instance pneumatic transport of the amoxicillin trihydrate from the separation step to the drying step. Without wishing to be bound by any scientific theory, it is believed that a limited extent of mechanical impact has the effect that relatively large crystals having the shape of needles are broken, thereby resulting in an increase of the bulk density and/or tapped density. However, a too high extent of mechanical forces is believed to result in the production of crystals that are too fine, thereby reducing the bulk density and/or tapped density. Using this insight provided by the invention and by varying the mechanical forces the skilled man is able to find out the conditions where the optimal bulk density and/or tapped density are achieved.

Accordingly, the invention provides a process for preparing crystalline amoxicillin trihydrate powder, said process comprising: crystallizing the amoxicillin trihydrate from a solution containing dissolved amoxicillin; separating the crystals from said solution; drying the separated crystals; wherein the process, preferably the crystallizing, separating and/or drying, is carried out under such conditions that the resulting crystalline powder has a bulk density higher than 0.45 g/ml, preferably higher than 0.5 g/ml, more preferably higher than 0.55 g/ml. There is no specific upper limit for the bulk density. The process, preferably the crystallizing, separating and/or drying, may for instance be carried out under such conditions that the bulk density is less than 0.8 g/ml, for instance less than 0.7 g/ml.

The invention also provides a process for preparing crystalline amoxicillin trihydrate powder, said process comprising: crystallizing amoxicillin trihydrate from a solution containing dissolved amoxicillin; separating the crystals from said solution; drying the separated crystals; wherein the process, preferably the crystallizing, separating and/or drying, is carried out under such conditions that the resulting crystalline powder has a tapped density higher than 0.6 g/ml, preferably higher than 0.7 g/ml, more preferably higher than 0.8 g/ml. There is no specific upper limit for the tapped density. The process, preferably the crystallizing, separating and/or drying, may for instance be carried out under such conditions that the tapped density is less than 1.2 g/ml, for instance less than 1.1 g/ml, for instance less than 1.0 g/ml.

The invention also provides a process for preparing crystalline amoxicillin trihydrate powder, said process comprising: crystallizing amoxicillin trihydrate from a solution containing dissolved amoxicillin; separating the crystals from said solution; drying the separated crystals; wherein the process, preferably the crystallizing, separating and/or drying, is carried out under such conditions that the resulting crystalline powder has ratio dₜ/d_{b} of less than 1.7, preferably less than 1.6, preferably less than 1.5, preferably less than 1.45, wherein dₜ = tapped density and d_{b} = bulk density. There is no specific lower limit for the ratio. The process, preferably the crystallizing, separating and/or drying, may for instance be carried out under such conditions that the ratio dₜ/d_{b} is higher than 1.05, for instance higher than 1.1.

The invention also provides a process for preparing crystalline amoxicillin trihydrate powder, said process comprising: crystallizing amoxicillin trihydrate from a solution containing dissolved amoxicillin; separating the crystals from said solution; drying the separated crystals; wherein the process, preferably the crystallizing, separating and/or drying, is carried out under such conditions that the compressibility index as defined by ((d, - d_{b})/dₜ)*100% is less than 40%, preferably less than 35%, more preferably less than 30%, wherein dₜ = tapped density and d_{b} = bulk density. There is no specific lower limit for the compressibility index. The compressibility index may for instance be higher than 10%.

The invention also provides a process for preparing crystalline amoxicillin trihydrate powder, said process comprising: crystallizing amoxicillin trihydrate from a solution containing dissolved amoxicillin; separating the crystals from said solution; drying the separated crystals; wherein the process, preferably the crystallizing, separating and/or drying, is carried out under such conditions that the resulting crystalline powder has a d₅₀ of higher than 10 µm, preferably higher than 20 µm, more preferably higher than 30 µm, in particular higher than 35 µm, more preferably higher than 40 µm. There is no specific upper limit for the d₅₀. The process, preferably the crystallizing, separating and/or drying, may for instance be carried out under such conditions the d₅₀ of the resulting crystalline powder is less than 150 µm, for instance less than 100 µm. Preferably, the process, preferably the crystallizing, separating and/or drying, is carried out under such conditions that the dried crystals have a d₁₀ of higher than 3 µm, preferably higher than 5 µm, more preferably higher than 8 µm, more preferably higher than 10 µm. There is no specific upper limit for d₁₀. The process, preferably the crystallizing, separating and/or drying, may be carried out under such conditions that d₁₀ of the resulting crystalline powder is less than 50 µm. The water activity that may be used to specify the extent to which a product is dried, may have any suitable value. Drying may be carried out such that the resulting crystalline amoxicillin trihydrate powder has a water activity of for instance higher than 0.05, for instance higher than 0.1, for instance higher than 0.15, for instance higher than 0.2, for instance higher than 0.25, for instance higher than 0.3, for instance less than 0.7, for instance less than 0.6, for instance less than 0.5. The water activity of the crystalline amoxicillin trihydrate powder according to the invention may for instance be higher than 0.05, for instance higher than 0.1, for instance higher than 0.15, for instance higher than 0.2, for instance higher than 0.25, for instance higher than 0.3. The water activity of the crystalline amoxicillin trihydrate powder according to the invention may for instance be less than 0.7, for instance less than 0.6, for instance less than 0.5. These values refer to the water activity measured at 25°C. The water activity is known to be defined as the equilibrium relative humidity divided by 100%. A preferred method for determining the water activity of a sample is to bring a quantity of the sample in a closed chamber having a relatively small volume, measuring the relative humidity as a function of time until the relative humidity has become constant (for instance after 30 minutes), the latter being the equilibrium relative humidity for that sample. Preferably, a Novasina TH200 Thermoconstanter is used, of which the sample holder has a volume of 12 ml and which is filled with 3 g. of sample.

Further preferred conditions for the preparation of crystalline amoxicillin trihydrate powder are disclosed hereinabove in the entire specification, including those conditions disclosed in the first part of the specification titled "amoxicillin trihydrate° and in all claims.

The invention will be further elucidated by means of the following examples, without however being limited thereto.

### Examples and Comparative experiment:

### Preparation of immobilized enzyme

*Escherichia coli* penicillin acylase was isolated as described in WO-A-9212782, purified using ion exchange chromatography, and immobilized as described in EP-A-222462 and WO-A-9704086.

As definition of penicillin G acylase activity the following is used: one unit (U) corresponds to the amount of enzyme that hydrolyses per minute 1 µmole penicillin G under standard conditions (100 g.l⁻¹ Penicillin G potassium salt, 0.05 M potassium phosphate buffer, pH value 8.0, 28 °C).

### Production of amoxicillin

162.2 g of 6-APA (6-amino-penicillanic acid) and 184.8 g of HPGM (D(-)-p-hydroxyphenylglycine methyl ester) were suspended in 450 ml of water. The suspension was cooled to a temperature of 10°C. To this reaction mixture 32850 Units of immobilized penicillin acylase were added, and water was added to a final volume of 1500 ml. The mixture was stirred for 6 hours. During the reaction the pH increased to 6.9, and at the end of the reaction the pH had decreased to 6.2. To this mixture 750 ml of water was added, and the suspension was filtrated over a sieve (with a mesh of 100 micrometer) in 2 hours to separate off the immobilized enzyme. The suspension obtained containing the amoxicillin trihydrate crystals was cooled to 0°C. The suspension contained less than 50 ppm of protein relative to amoxicillin trihydrate (less than 50 weight parts of protein per 1.000.000 weight parts of amoxicillin trihydrate).

An aqueous suspension containing amoxicillin in water (100 g amoxicillin trihydrate per liter of suspension) as obtained above was mixed with a 32 wt.% HCl solution (at a temperature of 25 °C) using a static mixer such as to obtain a solution having a pH of 1. The residence time in the static mixer was 1.5 minutes. The acidic solution obtained is pumped through two filters, the first filter having pores of 40 □m, the second filter having pores of 4.5 µm. The residence time in the filters was about 3 minutes. The acidic filtered solution and is fed to a first stirred tank in which a pH of 3.7 is maintained by addition of a 8 M NaOH solution. The temperature in the first tank is between 17 and 23 °C. The residence time in the first tank is 45 minutes. The contents of the first tank are fed to a second stirred tank in which a pH of 5.0 is maintained by addition of a 8 M NaOH solution. The temperature in the second stirred tank is between 17 and 23 °C. The residence time in the second stirred tank is 15 minutes. The contents of tank 2 fed to a third stirred tank in which the a temperature of 1 to 5 °C is maintained, the residence time in the third tank being more than 4 hours. The contents of the third stirred tank are fed to an inverted filter centrifuge such as to isolate the amoxicillin crystals, resulting in a wet cake containing 86 wt.% solid material. The wet cake was washed with water, pneumatically transported to a conical vacuum contact drier (Vrieco-Nauta), in which it was dried at a temperature of 30 to 40 °C and a pressure of 30 mbar during 7 hours.

### Measurement of particle size distribution.

The particle size distribution (including d₁₀ and d₅₀) was determined using a Malvern particle sizer 2600 C with objective f = 300 mm, malvern sample measurement unit PS1 and a Malvern dry powder feeder PS 64. The beam length was 14.30 mm. A polydisperse analysis model was used.

### Measurement of adsorption isotherms

Adsorption isotherms were determined using dynamic vapour sorption, using a VTI-SGA 100 vapour sorption analyser. Samples were used having a weight of 200 mg. Air inside the sample chamber was conditioned at a relative humidity of 10% for 90 minutes,. Subsequently, the relative humidity was increased with steps of 10%, while maintaining the sample was held at each value for the relative humidity for 90 minutes. The weight of the sample after said 90 minutes was taken as the sample weight corresponding to the equilibrium relative humidity. The temperature was 25 °C.

### Example I

A batch of amoxicillin trihydrate crystalline powder was prepared using the process as described above. The adsorption isotherm, as well as the particle size distribution was determined. The adsorption isotherm is shown in figure I. The d₅₀ and d₁₀ are indicated in the table.

The free water content, measured at an equilibrium relative humidity of 30% at 25 °C was 0.05 wt.%.

### Comparative experiment A

In a chemical process for the preparation of amoxicillin, a solution containing amoxicillin in dilute HCl and isopropanol was obtained. This solution was fed to a stirred tank. The pH was kept at 3.7 at a temperature of 20 °C. Subsequently the pH was raised to 5.0 by adding NaOH. The resulting mixture is maintained in a vessel at 1 to 5 °C during 3 to 12 hours. The amoxicillin was separated using a centrifuge, and dried using a fluid bed dryer.

The adsorption isotherm, as well as the particle size distribution was determined. The adsorption isotherm is shown in figure I. The d₅₀ and d₁₀ are indicated in the table.

The free water content, measured at an equilibrium relative humidity of 30% at 25 °C was 0.11 wt.%.

| | Bulk dens. (g/ml) | Tap. dens. (g/ml) | d₅₀ (µm) | d₁₀ (µm) | Free water content at ERH = 30% (in wt.%) |
|---|---|---|---|---|---|
| Ex. 1 | 0.51 | 0.73 | 43.1 | 11.4 | 0.05 wt.% |
| Comp. A | | | 8.3 | 2.7 | 0.11 wt.% |

Comparison of example I and comparative experiment A shows that the amoxicillin powder of example I contains less free water than the amoxicillin powder of comparative experiment A.

### Example II

Amoxicillin trihydrate powder obtained by the process of example I is dried to a water activity of 0.15. The powder is mixed with potassium clavulanate in a weight ratio 4 to 1 (calculated for the amoxicillin anhydrate and clavulanic acid). The mixture is stable.

### Example III

Amoxiallin powder obtained by the process of example I is dried to a water activity of 0.2. The powder is mixed with potassium clavulanate in a ratio 4 to 1. The mixture is stable.

### Example IV

Amoxicillin powder obtained by the process of example I is dried to a water activity of 0.15. The powder is mixed with potassium clavulanate in a ratio 4 to 1. The mixture is stable.

### Examples V-IX

Five different batches were prepared using the method described above. Table 2 shows the tapped density, bulk density, d₅₀, d₁₀ of the resulting crystals.

### Example X

The powder of example V was subjected to air jet sieving (200 LS-N air jet sieve manufactured by Hosakawa Alpine). Sieving was carried out using a 75 µm screen during 10 minutes. A few agglomerates formed during said sieving were removed from the overheads fraction (not passed through the sieve) tusing a vibrating sieve (425 µm), after which tapped density, bulk density, d₅₀, d₁₀ of the resulting crystals of the overheads fraction was determined. The results are indicated in table2. This example shows that the compressibility and the Hausner ratio are further decreased. The resulting powder flows through Klein cup 8 mm.

**Table 2**

| | Bulk dens. (d_{b}) (g/ml) | Tap. dens. (dₜ) (g/ml) | dₜ/d_{b} | d_{b}*(1/d_{b}-1/dₜ) *100% | d₅₀ (µm) | d₁₀ (µm) |
|---|---|---|---|---|---|---|
| Ex. V | 0.51 | 0.73 | 1.43 | 30% | 43.1 | 11.4 |
| Ex. VI | 0.56 | 0.78 | 1.39 | 28% | 42.8 | 8.8 |
| Ex. VII | 0.56 | 0.81 | 1.45 | 31% | 47.8 | 9.9 |
| Ex. VIII | 0.54 | 0.81 | 1.50 | 33% | 37.0 | 7.0 |
| Ex. IX | 0.58 | 0.79 | 1.36 | 26% | 61 | 19 |
| Ex. X | 0.59 | 0.74 | 1.25 | 20% | 86 | 36 |
| Comp. A | 0.26 | 0.55 | 2.12 | 53% | 17 | 4.0 |
| Ref. B | 0.26 | 0.47 | 1.8 | 45% | 66.3 | 17.4 |

### Comparative experiment A

In a chemical process for the preparation of amoxicillin, a solution containing amoxicillin in dilute HCl and isopropanol was obtained. This solution was fed to a stirred tank. The pH was kept at 3.7 at a temperature of 20 °C. Subsequently the pH was raised to 5.0 by adding NaOH. The resulting mixture is maintained in a vessel at 1 to 5 °C during 3 to 12 hours. The amoxicillin was separated using a centrifuge, and dried using a fluid bed dryer. Table 2shows the tapped density, bulk density, d₅₀, d₁₀.

### Reference experiment B

Example I was repeated with the difference that a the drying was not carried out using the a conical vacuum contact drier (Vrieco Nauta), but using a drier wherein the material was not mechanically impacted (Ventilation stove). Drying was carried out at a temperature of 35 °C during 16 hours. The results are indicated in table2. Comparison of examples I-IV with example B shows that the use of mechanical impact during drying results in an increased bulk density and tapped density.

### Example XI

6 Gelatine capsules (size 0) were filled by hand with of crystalline amoxicillin trihydrate powder of example IV (500 mg on an anhydrous basis per capsule). No auxiliaries were added. Dissolution tests were applied using apparatus 1 under the conditions as described in USP 27 under paragraph "amoxicillin capsules", including a stirring rate of 100 rpm and a temperature of 37 °C. Six beakers each containing 900 ml of water were used. Samples were taken each 5 minutes, and the quantity of dissolved amoxicillin were determined employing UV absorption at the wavelength of maximum absorbance at 272 nm.
84 wt.% of the amoxicillin was dissolved in 15 minutes.
90 wt.% of the amoxicillin was dissolved in 20 minutes.

### Comparative experiment C

Example VII was repeated using commercial granular auxiliary-free amoxicillin trihydrate granules obtained by roller compacting of amoxicillin trihydrate powder having the size distribution as indicated in table 3. After 60 minutes 82.0 wt.% of the amoxicillin was dissolved.

### Comparative experiment D

Example VII was repeated using commercial granular auxiliary-free amoxicillin trihydrate having a different particle size distribution as Indicated in table 3.. After 60 minutes 84.4 wt.% of the amoxicillin was dissolved.

**Table 3**

| | <125 µm | 125-250 µm | 250-500 µm | 500-850 µm | 850-1000 µm | >1000 µm | Bulk density | Tapped density |
|---|---|---|---|---|---|---|---|---|
| Ex. C | 14.5 | 4.9 | 20.7 | 53.7 | 5.6 | 0 | 0.68 | 0.85 |
| Ex. D | 8.6 | 3.1 | 24.9 | 57.1 | 6.3 | 0.3 | 0.69 | 0.83 |

### Example XII

20g of amoxicillin trihydrate crystalline powder according to the invention, 2 g of microcrystalline cellulose (Avicel^{®} PH102) were weighed in a 200 ml container and mixed in a Turbula T2C blender for 5 minutes. 0.1g of Magnesium stearate was added mixed for an additional 2 minutes.

Part of the mixture was transferred to a die with a diameter of 7 mm and a height of 2.3 cm, such that the die was completely filled. A punch was placed on top of the die and a pressure of 5 kg was applied during 5 seconds. The resulting empty space in the die was completely filled and a pressure of 5 kg was applied again. This procedure was repeated until no volume decrease was observed. Finally, the formed plug was released from the die in fed into an empty gelatine capsule body nr.0 Star-lock^{®} of Capsugel. The composition of the formulation is as follows:

| | |
|---|---|
| Amoxicillin trihydrate crystalline powder | 570.1mg (500mg amoxicillin) |
| Microcrystalline cellulose | 57.1 mg |
| Magnesium stearate | 2.8mg |
| Sum | 630.0 mg |

This example shows that the crystalline powder according to the invention can directly be used for filling capsules without compactation or granulation step in a specified quantity of 500 mg amoxicillin (calculated as amoxicillin anhydrate)

### Example IX

297.5g of amoxicillin trihydrate crystalline powder according to the invention, 88.5g of microcrystalline cellulose (Avicel^{®} PH200), 10g of croscarmellose sodium (Ac-Di-Sol^{®}), and 1g of silicium dioxide colloidal (Aerosil^{®} 200), are weighed in a 1000ml container and mixed in a Turbula TC2 blender for 10 minutes, 3g of magnesium stearate is added and mixed for an additional 2 min.

The mixture is transferred to a hopper of a Korsch^{®} EKO excenter tablet press, and tablets are pressed with the following properties:

The composition of the formulation is as follows:

| | |
|---|---|
| Amoxicillin trihydrate crystalline powder | 581.7mg (500mg amoxicillin) |
| Microcrystalline cellulose | 190.3mg |
| Croscarmellose sodium | 20 |
| Silicium dioxide colloidal | 2 |
| Magnesium stearate | 6 mg |
| | 800 mg |

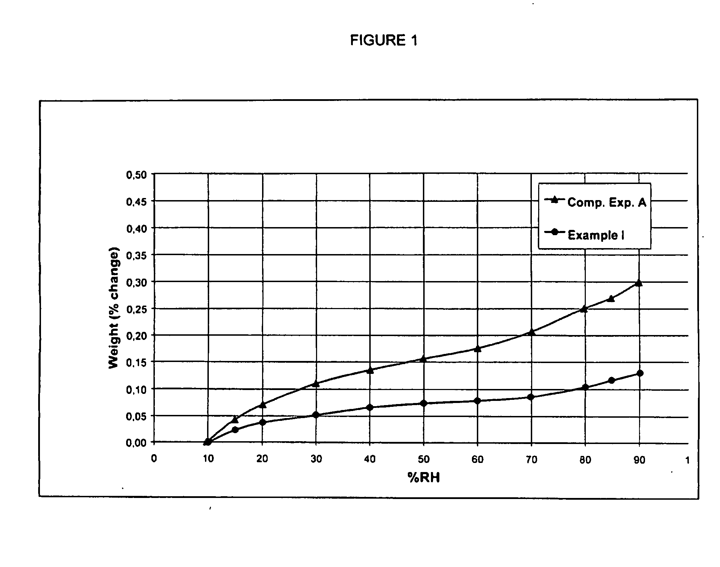

## Claims

1. Product of amoxicillin trihydrate, having a free water content of less than 0.1 wt.%, measured at an equilibrium relative humidity of 30% and at a temperature of 25 °C.

2. Product according to claim 1, wherein said product has a free water content of less than 0.07 wt.%, more preferably less than 0.05 wt.%, measured at an equilibrium relative humidity of 30% and at a temperature of 25 °C.

3. Product according to claim 1 or claim 2, having a water activity of more than 0.05, preferably more than 0.07, preferably more than 0.10, preferably more than 0.15, preferably more than 0.20, preferably more than 0.25, preferably more than 0.30, measured at a temperature of 25 °C.

4. Product according to any one of claims 1 to 3, wherein said product is crystalline amoxicillin trihydrate powder.

5. Crystalline powder according to claim 4, having a d₅₀ higher than 10 µm, preferably higher than 20 µm, more preferably higher than 30 µm, more preferably higher than 35 µm, more preferably higher than 40 µm.

6. Crystalline powder according to claim 4 or claim 5, having a d₁₀ of higher than 3 µm, preferably higher than 5 µm, more preferably higher than 8 µm, more preferably higher than 10 µm.

7. Crystalline powder according to any one of claims 4 to 6, having a bulk density higher than 0.45 g/ml, preferably higher than 0.5 g/ml, more preferably higher than 0.55 g/ml.

8. Crystalline powder according to any one of claims 4 to 7 having a tapped density higher than 0.6 g/ml, preferably higher than 0.7, more preferably higher than 0.8 g/ml.

9. Product according to any one of claims 1 to 8, wherein the product is crystalline powder according to any one of claims 22 to 26 or a product according to any one of claims 29 to 34.

10. Process comprising mixing the product according to any one of claims 1 to 9 with a second pharmaceutically active agent, and/or auxiliaries.

11. Mixture comprising
(i) the product according to any one of claims 1 to 9; and
(ii) a second pharmaceutically active agent, and/or auxiliaries.

12. Process according to claim 10 or mixture according to claim 11, wherein said second pharmaceutically active agent is clavulanic acid in the form of a salt, preferably clavulanic acid in the form of a potassium salt.

13. Process comprising compressing the product according to any one of claims 1 to 9 or the mixture according to claim 11 to produce compressed products.

14. Process according to claim 13, wherein said compressed products are granules or tablets.

15. Use of the product according to any one of claims 1 to 9 for the preparation of a pharmaceutical composition.

16. Use of the product according to any one of claims 1 to 9 or the mixture according to claim 10 for the preparation of a tablet or for filling a capsule.

17. Process for preparing amoxicillin trihydrate, said process comprising:
preparing amoxicillin by reacting 6-amino-penicillanic acid or a salt thereof, with para-hydroxyphenyl glycine in activated form in the presence of an enzyme immobilized on a carrier;
forming an aqueous solution containing the amoxicillin, said aqueous solution containing hydrochloric acid; and
crystallizing amoxicillin trihydrate from said aqueous solution.

18. Process according to claim 17, wherein the aqueous solution from which the amoxicillin is crystallized has an amoxicillin concentration of less than 0.6 mol/l, preferably less than 0.5 mol/l, more preferably less than 0.4 mol/l, more preferably less than 0.3 mol/l.

19. Process according to claim 17 or claim 18, wherein the solution from which the amoxicillin is crystallized contains less than 200 weight parts of protein per 1.000.000 weight parts of the amoxicillin in said solution, preferably less 100 weight parts of protein, more preferably less than 50 weight parts of protein, more preferably less than 35 weight parts of protein.

20. Process according to any one of claims 17 to 19, wherein the process comprises separating the crystals from said aqueous solution; and drying the separated crystals, resulting in crystalline powder having a d₅₀ higher than 10 µm, preferably higher than 20 µm, more preferably higher than 30 µm, more preferably higher than 35 µm, more preferably higher than 40 µm.

21. Process according to any one of claims 17 to 20, resulting in crystalline powder having a d₁₀ higher than 3 µm, preferably higher than 5 µm, more preferably higher than 8 µm, more preferably higher than 10 µm.

22. Crystalline amoxicillin trihydrate powder having a bulk density higher than 0.45 g/ml, preferably higher than 0.5 g/ml, more preferably higher than 0.55 g/ml.

23. Crystalline powder according to claim 22, having a tapped density higher than 0.6 g/ml, preferably higher than 0.7, more preferably higher than 0.8 g/ml.

24. Crystalline powder according to claim 22 or claim 23, having a d₅₀ higher than 10 µm, preferably higher than 20 µm, more preferably higher than 30 µm, more preferably higher than 35 µm, more preferably higher than 40 µm, preferably lower than 150 µm.

25. Crystalline powder according to any one of claims 22 to 24, having a d₁₀ higher than 3 µm, preferably higher than 5 µm, more preferably higher than 8 µm, more preferably higher than 10 µm.

26. Crystalline powder according to any one of claims 22 to 25, having a T_{85%} < 55 minutes, preferably less than 50 minutes, preferably less than 40 minutes, preferably lesst than 30 minutes, wherein T_{85%} is the period required to effect dissolution of 85 wt.% of a predetermined quantity (500 mg on an anhydrous basis) of the amoxicillin trihydrate in 900 ml water of 37 °C.

27. Crystalline powder according to any one of claims 22 to 26, wherein the crystalline powder is crystalline powder according to any one of claims 3 to 8.

28. Mixture comprising
(i) crystalline powder according to any one of claims 22 to 27; and
(ii)
(a) granules comprising amoxicillin trihydrate; and/or
(b) a second pharmaceutically active agent; and/or
(c) auxiliaries

29. Product of amoxicillin trihydrate having
(i) T_{85%} < 55 minutes, preferably < 50 minutes, preferably < 40 minutes, preferably < 30 minutes, preferably < 25 minutes; and
(ii) bulk density higher than 0.45 g/ml, preferably higher than 0.5 g/ml, more preferably higher than 0.55 g/ml.

30. Product according to claim 29, comprising (i) amoxicillin trihydrate powder and (ii) granules comprising amoxicillin.

31. Product according to claim 30, wherein said amoxicillin trihydrate powder is crystalline amoxicillin trihydrate powder according to any one of claims 22 to 26.

32. Product according to claim 30 or claim 31, wherein said granules have a d₅₀ of between 100 and 1000 µm.

33. Product according to any one of claims 30 to 32 or a mixture according to claim 26, wherein said granules are free of auxiliaries.

34. Product according to any one of claims 29 to 33, wherein said product is free of auxiliaries.

35. Mixture comprising
(i) product according to any one of claims 29 to 34 and
(ii) auxiliaries and/or a second pharmaceutically active agent.

36. Mixture according to claim 35, said mixture having:
(i) T_{85%} < 60 minutes, preferably < 50 minutes, preferably < 40 minutes, preferably < 30 minutes, preferably < 25 minutes; and
(ii) bulk density higher than 0.45 g/ml, preferably higher than 0.5 g/ml, more preferably higher than 0.55 g/ml.

37. Mixture according to claim 28 or claim 35 or claim 36, wherein said second pharmaceutically active agent is clavulanic acid in the form of a salt, preferably clavulanic acid in the form of a potassium salt.

38. Use of the crystalline powder according to any one of claims 22 to 27 or the product according to any one of claims 29 to 34 or a mixture according to claim 28 or any one of claims 35 to 37 for filling a capsule or for the preparation of a tablet.

39. Capsule containing crystalline powder according to any one of claims 22 to 27 or the product according to any one of claims 29 to 34 or a mixture according to claim 28 or any one of claims 35 to 37.

40. Process comprising compressing crystalline powder according to any one of claims 22 to 27 or the product according to any one of claims 29 to 34 or a mixture according to claim 28 or any one of claims 35 to 37 to produce compressed products

41. Granules or tablets comprising the crystalline powder according to any one of claims 22 to 27 or the product according to any one of claims 29 to 34 or a mixture according to claim 28 or any one of claims 35 to 37 in compressed form.

42. Use of the crystalline powder according to any one of claims 22 to 27 or the product according to any one of claims 29 to 34 for the preparation of a pharmaceutical composition.

43. Process for preparing crystalline amoxicillin trihydrate powder, said process comprising:
crystallizing amoxicillin trihydrate from a solution containing dissolved amoxicillin;
separating the crystals from said solution; and
drying the separated crystals;
resulting in crystalline powder having a bulk density higher than 0.45 g/ml, preferably higher than 0.5 g/ml, more preferably higher than 0.55 g/ml.

44. Process according to claim 43, resulting in crystalline powder having a tapped density higher than 0.6 g/ml, preferably higher than 0.7 g/ml, more preferably higher than 0.8 g/ml.

45. Process according to claim 43 or claim 44, resulting in crystalline powder having a d₅₀ higher than 10 µm, preferably higher than 20 µm, more preferably higher than 30 µm, more preferably higher than 35 µm, more preferably higher than 40 µm.

46. Process according to any one of claims 43 to 45, resulting in crystalline powder having a d₁₀ higher than 3 µm, preferably higher than 5 µm, more preferably higher than 8 µm, more preferably higher than 10 µm.

47. Process according to any one of claims 43 to 46, wherein the process comprises mechanically impacting the crystals during said drying.

48. Process according to any one of claims 43 to 47, wherein the process comprises steps of the process according to any one of claims 17 to 21.

## Patentansprüche

1. Amoxicillin-trihydrat-Produkt mit einem Gehalt an freiem Wasser von weniger als 0,1 Gew.-%, gemessen bei einer relativen Gleichgewichtsfeuchtigkeit von 30% und bei einer Temperatur von 25°C.

2. Produkt nach Anspruch 1, wobei das Produkt einen Gehalt an freiem Wasser von weniger als 0,07 Gew.-%, besonders bevorzugt weniger von 0,05 Gew.-%, gemessen bei einer relativen Gleichgewichtsfeuchtigkeit von 30% und bei einer Temperatur von 25°C, aufweist.

3. Produkt nach Anspruch 1 oder 2 mit einer Wasseraktivität von mehr als 0,05, vorzugsweise mehr als 0,07, vorzugsweise mehr als 0,10, vorzugsweise mehr als 0,15, vorzugsweise mehr als 0,20, vorzugsweise mehr als 0,25, vorzugsweise mehr als 0,30, gemessen bei einer Temperatur von 25°C.

4. Produkt nach einem der Ansprüche 1 bis 3, wobei es sich bei dem Produkt um kristallines Amoxicillin-trihydrat-Pulver handelt.

5. Kristallines Pulver nach Anspruch 4 mit einem d₅₀ von mehr als 10 µm, vorzugsweise mehr als 20 µm, weiter bevorzugt mehr als 30 µm, weiter bevorzugt mehr als 35 µm, weiter bevorzugt mehr als 40 µm.

6. Kristallines Pulver nach Anspruch 4 oder 5 mit einem d₁₀ von mehr als 3 µm, vorzugsweise mehr als 5 µm, weiter bevorzugt mehr als 8 µm, weiter bevorzugt mehr als 10 µm.

7. Kristallines Pulver nach einem der Ansprüche 4 bis 6 mit einer Schüttdichte von mehr als 0,45 g/ml, vorzugsweise mehr als 0,5 g/ml, weiter bevorzugt mehr als 0,55 g/ml.

8. Kristallines Pulver nach einem der Ansprüche 4 bis 7 mit einer Stampfdichte von mehr als 0,6 g/ml, vorzugsweise mehr als 0,7, weiter bevorzugt mehr als 0,8 g/ml.

9. Produkt nach einem der Ansprüche 1 bis 8, wobei es sich bei dem Produkt um ein kristallines Pulver nach einem der Ansprüche 22 bis 26 oder ein Produkt nach einem der Ansprüche 29 bis 34 handelt.

10. Verfahren, bei dem man das Produkt nach einem der Ansprüche 1 bis 9 mit einem zweiten pharmazeutischen Wirkstoff und/oder Hilfsstoffen mischt.

11. Mischung, enthaltend:
(i) das Produkt nach einem der Ansprüche 1 bis 9; und
(ii) einen zweiten pharmazeutischen Wirkstoff und/oder Hilfsstoffe.

12. Verfahren nach Anspruch 10 oder Mischung nach Anspruch 11, wobei es sich bei dem zweiten pharmazeutischen Wirkstoff um Clavulansäure in Form eines Salzes, vorzugsweise Clavulansäure in Form eines Kaliumsalzes, handelt.

13. Verfahren, bei dem man das Produkt nach einem der Ansprüche 1 bis 9 oder die Mischung nach Anspruch 11 zur Herstellung von komprimierten Produkten komprimiert.

14. Verfahren nach Anspruch 13, wobei es sich bei den komprimierten Produkten um Granulate oder Tabletten handelt.

15. Verwendung des Produkts nach einem der Ansprüche 1 bis 9 zur Herstellung einer pharmazeutischen Zusammensetzung.

16. Verwendung des Produkts nach einem der Ansprüche 1 bis 9 oder der Mischung nach Anspruch 10 zur Herstellung einer Tablette oder zum Füllen einer Kapsel.

17. Verfahren zur Herstellung von Amoxicillin-trihydrat, wobei man bei dem Verfahren:
Amoxicillin herstellt, indem man 6-Aminopenicillansäure oder ein Salz davon in Gegenwart eines an einem Träger immobilisierten Enzyms mit para-Hydroxyphenylglycin in aktivierter Form umsetzt;
eine wäßrige, das Amoxicillin enthaltende Lösung bildet, wobei die wäßrige Lösung Salzsäure enthält; und
aus dieser wäßrigen Lösung Amoxicillin-trihydrat kristallisiert.

18. Verfahren nach Anspruch 17, wobei die wäßrige Lösung, aus der das Amoxicillin kristallisiert wird, eine Amoxicillinkonzentration von weniger als 0,6 mol/l, vorzugsweise weniger als 0,5 mol/l, weiter bevorzugt weniger als 0,4 mol/l, weiter bevorzugt weniger als 0,3 mol/l, aufweist.

19. Verfahren nach Anspruch 17 oder 18, wobei die Lösung, aus der das Amoxicillin kristallisiert wird, weniger als 200 Gewichtsteile Protein pro 1.000.000 Gewichtsteile Amoxicillin in dieser Lösung, vorzugsweise weniger als 100 Gewichtsteile Protein, weiter bevorzugt weniger als 50 Gewichtsteile Protein, weiter bevorzugt weniger als 35 Gewichtsteile Protein, aufweist.

20. Verfahren nach einem der Ansprüche 17 bis 19, wobei man bei dem Verfahren die Kristalle aus der wäßrigen Lösung abtrennt und die abgetrennten Kristalle trocknet, wodurch man ein kristallines Pulver mit einem d₅₀ von mehr als 10 µm, vorzugsweise mehr als 20 µm, weiter bevorzugt mehr als 30 µm, weiter bevorzugt mehr als 35 µm, weiter bevorzugt mehr als 40 µm, erhält.

21. Verfahren nach einem der Ansprüche 17 bis 20, bei dem man ein kristallines Pulver mit einem d₁₀ von mehr als 3 µm, vorzugsweise mehr als 5 µm, weiter bevorzugt mehr als 8 µm, weiter bevorzugt mehr als 10 µm, erhält.

22. Kristallines Amoxicillin-trihydrat-Pulver mit einer Schüttdichte von mehr als 0,45 g/ml, vorzugsweise mehr als 0,5 g/ml, besonders bevorzugt mehr als 0,55 g/ml.

23. Kristallines Pulver nach Anspruch 22 mit einer Stampfdichte von mehr als 0,6 g/ml, vorzugsweise mehr als 0,7, weiter bevorzugt mehr als 0,8 g/ml.

24. Kristallines Pulver nach Anspruch 22 oder 23 mit einem d₅₀ von mehr als 10 µm, vorzugsweise mehr als 20 µm, weiter bevorzugt mehr als 30 µm, weiter bevorzugt mehr als 35 µm, weiter bevorzugt mehr als 40 µm, vorzugsweise weniger als 150 µm.

25. Kristallines Pulver nach einem der Ansprüche 22 bis 24 mit einem d₁₀ von mehr als 3 µm, vorzugsweise mehr als 5 µm, weiter bevorzugt mehr als 8 µm, weiter bevorzugt mehr als 10 µm.

26. Kristallines Pulver nach einem der Ansprüche 22 bis 25 mit einer T_{85%} < 55 Minuten, vorzugsweise weniger als 50 Minuten, vorzugsweise weniger als 40 Minuten, vorzugsweise weniger als 30 Minuten, wobei es sich bei T_{85%} um die Zeitspanne handelt, die erforderlich ist, um 85 Gew.-% einer vorbestimmten Menge (500 mg auf wasserfreier Basis) des Amoxicillin-trihydrats in 900 ml 37°C warmen Wassers zu lösen.

27. Kristallines Pulver nach einem der Ansprüche 22 bis 26, wobei es sich bei dem kristallinen Pulver um ein kristallines Pulver nach einem der Ansprüche 3 bis 8 handelt.

28. Mischung, enthaltend:
(i) ein kristallines Pulver nach einem der Ansprüche 22 bis 27; und
(ii)
(a) ein Granulat, das Amoxicillin-trihydrat enthält; und/oder
(b) einen zweiten pharmazeutischen Wirkstoff; und/oder
(c) Hilfsstoffe.

29. Amoxicillin-trihydrat-Produkt mit
(i) einer T_{85%} < 55 Minuten, vorzugsweise < 50 Minuten, vorzugsweise < 40 Minuten, vorzugsweise < 30 Minuten, vorzugsweise < 25 Minuten; und
(ii) einer Schüttdichte von mehr als 0,45 g/ml, vorzugsweise mehr als 0,5 g/ml, weiter bevorzugt mehr als 0,55 g/ml.

30. Produkt nach Anspruch 29, enthaltend (i) Amoxicillin-trihydrat-Pulver und (ii) ein Amoxicillin enthaltendes Granulat.

31. Produkt nach Anspruch 30, wobei es sich bei dem Amoxicillin-trihydrat-Pulver um kristallines Amoxicillin-trihydrat-Pulver nach einem der Ansprüche 22 bis 26 handelt.

32. Produkt nach Anspruch 30 oder 31, wobei das Granulat einen d₅₀ zwischen 100 und 1.000 µm aufweist.

33. Produkt nach einem der Ansprüche 30 bis 32 oder Mischung nach Anspruch 26, wobei das Granulat frei von Hilfsstoffen ist.

34. Produkt nach einem der Ansprüche 29 bis 33, wobei das Produkt frei von Hilfsstoffen ist.

35. Mischung, enthaltend:
(i) das Produkt nach einem der Ansprüche 29 bis 34 und
(ii) Hilfsstoffe und/oder einen zweiten pharmazeutischen Wirkstoff.

36. Mischung nach Anspruch 35, wobei die Mischung eine:
(i) T_{85%} < 60 Minuten, vorzugsweise < 50 Minuten, vorzugsweise < 40 Minuten, vorzugsweise < 30 Minuten, vorzugsweise < 25 Minuten; und eine
(ii) Schüttdichte von mehr als 0,45 g/ml, vorzugsweise mehr als 0,5 g/ml, besonders bevorzugt mehr als 0,55 g/ml, aufweist.

37. Mischung nach Anspruch 28 oder Anspruch 35 oder Anspruch 36, wobei es sich bei dem zweiten pharmazeutischen Wirkstoff um Clavulansäure in Form eines Salzes, vorzugsweise Clavulansäure in Form eines Kaliumsalzes, handelt.

38. Verwendung des kristallinen Pulvers nach einem der Ansprüche 22 bis 27 oder des Produkts nach einem der Ansprüche 29 bis 34 oder einer Mischung nach Anspruch 28 oder einem der Ansprüche 35 bis 37 zum Füllen einer Kapsel oder zur Herstellung einer Tablette.

39. Kapsel, enthaltend ein kristallines Pulver nach einem der Ansprüche 22 bis 27 oder das Produkt nach einem der Ansprüche 29 bis 34 oder eine Mischung nach Anspruch 28 oder einem der Ansprüche 35 bis 37.

40. Verfahren, bei dem man zur Herstellung von komprimierten Produkten ein kristallines Pulver nach einem der Ansprüche 22 bis 27 oder das Produkt nach einem der Ansprüche 29 bis 34 oder eine Mischung nach Anspruch 28 oder einem der Ansprüche 35 bis 37 komprimiert.

41. Granulat oder Tabletten, enthaltend das kristalline Pulver nach einem der Ansprüche 22 bis 27 oder das Produkt nach einem der Ansprüche 29 bis 34 oder eine Mischung nach Anspruch 28 oder einem der Ansprüche 35 bis 37 in komprimierter Form.

42. Verwendung des kristallinen Pulvers nach einem der Ansprüche 22 bis 27 oder des Produkts nach einem der Ansprüche 29 bis 34 zur Herstellung einer pharmazeutischen Zusammensetzung.

43. Verfahren zur Herstellung von kristallinem Amoxicillin-trihydrat-Pulver, wobei man bei dem Verfahren:
Amoxicillin-trihydrat aus einer gelöstes Amoxicillin enthaltenden Lösung kristallisiert; die Kristalle aus dieser Lösung abtrennt; und die abgetrennten Kristalle trocknet;
wodurch man ein kristallines Pulver mit einer Schüttdichte von mehr als 0,45 g/ml, vorzugsweise mehr als 0,5 g/ml, weiter bevorzugt mehr als 0,55 g/ml, erhält.

44. Verfahren nach Anspruch 43, bei dem man ein kristallines Pulver mit einer Stampfdichte von mehr als 0,6 g/ml, vorzugsweise mehr als 0,7 g/ml, weiter bevorzugt mehr als 0,8 g/ml, erhält.

45. Verfahren nach Anspruch 43 oder 44, bei dem man ein kristallines Pulver mit einem d₅₀ von mehr als 10 µm, vorzugsweise mehr als 20 µm, weiter bevorzugt mehr als 30 µm, weiter bevorzugt mehr als 35 µm, weiter bevorzugt mehr als 40 µm, erhält.

46. Verfahren nach einem der Ansprüche 43 bis 45, bei dem man ein kristallines Pulver mit einem d₁₀ von mehr als 3 µm, vorzugsweise mehr als 5 µm, weiter bevorzugt mehr als 8 µm, weiter bevorzugt mehr als 10 µm, erhält.

47. Verfahren nach einem der Ansprüche 43 bis 46, wobei man bei dem Verfahren die Kristalle während der Trocknung mechanisch stößt.

48. Verfahren nach einem der Ansprüche 43 bis 47, wobei das Verfahren die Schritte des Verfahrens nach einem der Ansprüche 17 bis 21 umfaßt.

## Revendications

1. Produit à base de trihydrate d'amoxicilline, ayant une teneur en eau libre inférieure à 0,1 % en poids, mesurée à une humidité relative d'équilibre de 30 % et à une température de 25 °C.

2. Produit selon la revendication 1, dans lequel ledit produit a une teneur en eau libre inférieure à 0,07 % en poids, plus préférablement inférieure à 0,05 % en poids, mesurée à une humidité relative d'équilibre de 30 % et à une température de 25 °C.

3. Produit selon l'une des revendications 1 ou 2, qui possède une activité d'eau supérieure à 0,05, de préférence supérieure à 0,07, de préférence supérieure à 0,10, de préférence supérieure à 0,15, de préférence supérieure à 0,20, de préférence supérieure à 0,25, de préférence supérieure à 0,30, mesurée à une température de 25 °C.

4. Produit selon l'une quelconque des revendications 1 à 3, dans lequel ledit produit est du trihydrate d'amoxicilline sous forme d'une poudre cristalline.

5. Poudre cristalline selon la revendication 4, qui possède un d₅₀ supérieur à 10 µm, de préférence supérieur à 20 µm, plus préférablement supérieur à 30 µm, plus préférablement supérieur à 35 µm, plus préférablement supérieur à 40 µm.

6. Poudre cristalline selon l'une des revendications 4 ou 5, qui possède un d₁₀ supérieur à 3 µm, de préférence supérieur à 5 µm, plus préférablement supérieur à 8 µm, plus préférablement supérieur à 10 µm.

7. Poudre cristalline selon l'une quelconque des revendications 4 à 6, qui possède une masse volumique apparente supérieure à 0,45 g/ml, de préférence supérieure à 0,5 g/ml, plus préférablement supérieure à 0,55 g/ml.

8. Poudre cristalline selon l'une quelconque des revendications 4 à 7, qui possède une masse volumique après tassement supérieure à 0,6 g/ml, de préférence supérieure à 0,7 g/ml, plus préférablement supérieure à 0,8 g/ml.

9. Produit selon l'une quelconque des revendications 1 à 8, dans lequel le produit est une poudre cristalline selon l'une quelconque des revendications 22 à 26 ou un produit selon l'une quelconque des revendications 29 à 34.

10. Procédé comprenant le mélange du produit selon l'une quelconque des revendications 1 à 9 avec un second agent pharmaceutiquement actif et/ou des auxiliaires.

11. Mélange comprenant
(i) le produit selon l'une quelconque des revendications 1 à 9 ; et
(ii) un second agent pharmaceutiquement actif et/ou des auxiliaires.

12. Procédé selon la revendication 10 ou mélange selon la revendication 11, dans lequel ledit second agent pharmaceutiquement actif est de l'acide clavulanique sous la forme d'un sel, de préférence de l'acide clavulanique sous la forme d'un sel de potassium.

13. Procédé comprenant la compression du produit selon l'une quelconque des revendications 1 à 9 ou du mélange selon la revendication 11 pour produire des produits comprimés.

14. Procédé selon la revendication 13, dans lequel lesdits produits comprimés sont des granules ou des comprimés.

15. Utilisation du produit selon l'une quelconque des revendications 1 à 9 pour la préparation d'une composition pharmaceutique.

16. Utilisation du produit selon l'une quelconque des revendications 1 à 9 ou du mélange selon la revendication 10 pour la préparation d'un comprimé ou pour le remplissage d'une capsule.

17. Procédé pour la préparation de trihydrate d'amoxicilline, ledit procédé comprenant :
la préparation d'amoxicilline en faisant réagir de l'acide 6-aminopénicillanique, ou un sel de celui-ci, avec de la para-hydroxyphénylglycine sous une forme activée en présence d'une enzyme immobilisée sur un vecteur ;
la formation d'une solution aqueuse comprenant l'amoxicilline, ladite solution aqueuse comprenant de l'acide chlorhydrique ; et
la cristallisation du trihydrate d'amoxicilline à partir de ladite solution aqueuse.

18. Procédé selon la revendication 17, dans lequel la solution aqueuse à partir de laquelle l'amoxicilline est cristallisée présente une concentration en amoxicilline inférieure à 0,6 mole/l, de préférence inférieure à 0,5 mole/l, plus préférablement inférieure à 0,4 mole/l, plus préférablement inférieure à 0,3 mole/l.

19. Procédé selon l'une des revendications 17 ou 18, dans lequel la solution à partir de laquelle l'amoxicilline est cristallisée comprend moins de 200 parties en poids de protéines par 1 000 000 parties en poids d'amoxicilline dans ladite solution, de préférence moins de 100 parties en poids de protéines, plus préférablement moins de 50 parties en poids de protéines, plus préférablement moins de 35 parties en poids de protéines.

20. Procédé selon l'une quelconque des revendications 17 à 19, dans lequel le procédé comprend la séparation des cristaux de ladite solution aqueuse et le séchage des cristaux séparés, donnant une poudre cristalline qui possède un d₅₀ supérieur à 10 µm, de préférence supérieur à 20 µm, plus préférablement supérieur à 30 µm, plus préférablement supérieur à 35 µm, plus préférablement supérieur à 40 µm.

21. Procédé selon l'une des revendications 17 à 20, qui donne une poudre cristalline qui possède un d₁₀ supérieur à 3 µm, de préférence supérieur à 5 µm, plus préférablement supérieur à 8 µm, plus préférablement supérieur à 10 µm.

22. Trihydrate d'amoxicilline sous forme d'une poudre cristalline qui possède une masse volumique apparente supérieure à 0,45 g/ml, de préférence supérieure à 0,5 g/ml, plus préférablement supérieure à 0,55 g/ml.

23. Poudre cristalline selon la revendication 22, qui possède une masse volumique après tassement supérieure à 0,6 g/ml, de préférence supérieure à 0,7 g/ml, plus préférablement supérieure à 0,8 g/ml.

24. Poudre cristalline selon l'une des revendications 22 ou 23, qui possède un d₅₀ supérieur à 10 µm, de préférence supérieur à 20 µm, plus préférablement supérieur à 30 µm, plus préférablement supérieur à 35 µm, plus préférablement supérieur à 40 µm, plus préférablement inférieur à 150 µm.

25. Poudre cristalline selon l'une quelconque des revendications 22 à 24, qui possède un d₁₀ supérieur à 3 µm, de préférence supérieur à 5 µm, plus préférablement supérieur à 8 µm, plus préférablement supérieur à 10 µm.

26. Poudre cristalline selon l'une quelconque des revendications 22 à 25, qui possède un T_{85 %} inférieur à 55 minutes, de préférence inférieur à 50 minutes, de préférence inférieur à 40 minutes, de préférence inférieur à 30 minutes, dans laquelle T_{85%} représente la période requise pour obtenir la dissolution de 85 % en poids d'une quantité prédéterminée (500 mg sur une base anhydre) de trihydrate d'amoxicilline dans 900 ml d'eau à 37 °C.

27. Poudre cristalline selon l'une quelconque des revendications 22 à 26, dans laquelle la poudre cristalline est une poudre cristalline selon l'une quelconque des revendications 3 à 8.

28. Mélange comprenant
(i) une poudre cristalline selon l'une quelconque des revendications 22 à 27 ; et
(ii)
(a) des granules comprenant du trihydrate d'amoxicilline ; et/ou
(b) un second agent pharmaceutiquement actif ; et/ou
(c) des auxiliaires

29. Produit à base de trihydrate d'amoxicilline ayant
(i) un T_{85 %} inférieur à 55 minutes, de préférence inférieur à 50 minutes, de préférence inférieur à 40 minutes, de préférence inférieur à 30 minutes, de préférence inférieur à 25 minutes ; et
(ii) une masse volumique apparente supérieure à 0,45 g/ml, de préférence supérieure à 0,5 g/ml, plus préférablement supérieure à 0,55 g/ml.

30. Produit selon la revendication 29, qui comprend (i) du trihydrate d'amoxicilline en poudre et (ii) des granules qui comprennent de l'amoxicilline.

31. Produit selon la revendication 30, dans lequel ledit trihydrate d'amoxicilline en poudre est du trihydrate d'amoxicilline sous forme de poudre cristalline selon l'une quelconque des revendications 22 à 26.

32. Produit selon l'une des revendications 30 ou 31, dans lequel lesdits granules ont un d₅₀ situé entre 100 et 1000 µm.

33. Produit selon l'une quelconque des revendications 30 à 32 ou un mélange selon la revendication 26, dans lequel lesdits granules sont exempts d'auxiliaires.

34. Produit selon l'une quelconque des revendications 29 à 33, dans lequel ledit produit est exempt d'auxiliaires.

35. Mélange comprenant
(i) le produit selon l'une quelconque des revendications 29 à 34 et
(ii) des auxiliaires et/ou un second agent pharmaceutiquement actif.

36. Mélange selon la revendication 35, ledit mélange comprenant :
(i) un T_{85 %} inférieur à 60 minutes, de préférence inférieur à 50 minutes, de préférence inférieur à 40 minutes, de préférence inférieur à 30 minutes, de préférence inférieur à 25 minutes ; et
(ii) une masse volumique apparente supérieure à 0,45 g/ml, de préférence supérieure à 0,5 g/ml, plus préférablement supérieure à 0,55 g/ml.

37. Mélange selon l'une des revendications 28, 35 ou 36, dans lequel ledit second agent pharmaceutiquement actif est de l'acide clavulanique sous la forme d'un sel, de préférence de l'acide clavulanique sous la forme d'un sel de potassium.

38. Utilisation de la poudre cristalline selon l'une quelconque des revendications 22 à 27 ou du produit selon l'une quelconque des revendications 29 à 34 ou d'un mélange selon la revendication 28 ou l'une quelconque des revendications 35 à 37 pour le remplissage d'une capsule ou pour la préparation d'un comprimé.

39. Capsule comprenant la poudre cristalline selon l'une quelconque des revendications 22 à 27 ou le produit selon l'une quelconque des revendications 29 à 34 ou un mélange selon la revendication 28 ou l'une quelconque des revendications 35 à 37.

40. Procédé comprenant la compression de la poudre cristalline selon l'une quelconque des revendications 22 à 27 ou du produit selon l'une quelconque des revendications 29 à 34 ou du mélange selon la revendication 28 ou l'une quelconque des revendications 35 à 37 pour produire des produits comprimés.

41. Granules ou comprimés comprenant la poudre cristalline selon l'une quelconque des revendications 22 à 27 ou le produit selon l'une quelconque des revendications 29 à 34 ou un mélange selon la revendication 28 ou l'une quelconque des revendications 35 à 37 une forme comprimée.

42. Utilisation de la poudre cristalline selon l'une quelconque des revendications 22 à 27 ou du produit selon l'une quelconque des revendications 29 à 34 pour la préparation d'une composition pharmaceutique.

43. Procédé pour la préparation de trihydrate d'amoxicilline sous forme de poudre cristalline, ledit procédé comprenant :
la cristallisation du trihydrate d'amoxicilline à partir d'une solution comprenant l'amoxicilline dissoute ;
la séparation des cristaux de ladite solution ; et
le séchage des cristaux séparés ;
donnant une poudre cristalline qui possède une masse volumique apparente supérieure à 0,45 g/ml, de préférence supérieure à 0,5 g/ml, plus préférablement supérieure à 0,55 g/ml.

44. Procédé selon la revendication 43, qui donne une poudre cristalline qui possède une masse volumique après tassement supérieure à 0,6 g/ml, de préférence supérieure à 0,7 g/ml, plus préférablement supérieure à 0,8 g/ml.

45. Procédé selon l'une des revendications 43 ou 44, qui donne une poudre cristalline qui possède un d₅₀ supérieur à 10 µm, de préférence supérieur à 20 µm, plus préférablement supérieur à 30 µm, plus préférablement supérieur à 35 µm, plus préférablement supérieur à 40 µm.

46. Procédé selon l'une quelconque des revendications 43 à 45, qui donne une poudre cristalline qui possède un d₁₀ supérieur à 3 µm, de préférence supérieur à 5 µm, plus préférablement supérieur à 8 µm, plus préférablement supérieur à 10 µm.

47. Procédé selon l'une quelconque des revendications 43 à 46, dans lequel le procédé comprend la soumission des cristaux à un tassage mécanique pendant ledit séchage.

48. Procédé selon l'une quelconque des revendications 43 à 47, dans lequel le procédé comprend des étapes du procédé selon l'une quelconque des revendications 17 à 21.
